# EUROPEAN PATENT APPLICATION

(11) **EP 2 529 738 A1**
(43) Date of publication of application: **05.12.2012**
(21) Application number: 11185912.0
(22) Date of filing: 20.10.2011
(51) Int. Cl.: A61K 31/435, C07D 213/81, C07D 213/84, C07D 401/14, A61P 9/04

(54) **Substituted terpyridines**

(30) Priority: 29.05.2011 EP 11168008
(71) Applicant: Max-Delbrück-Centrum für Molekulare Medizin, 13125 Berlin (DE); Forschungsverbund Berlin e.V., 12489 Berlin (DE)
(72) Inventor: Schäfer, Gesa, 10439 Berlin (DE); Krause, Gerd, 13156 Berlin (DE); Rosenthal, Walter, 14153 Kleinmachnow (DE); Milic, Jelena, 13158 Berlin (DE); Rademann, Jörg, 13465 Berlin (DE); Schillinger, Christian, 10407 Berlin (DE); Klussmann, Enno, 14163 Berlin (DE)
(74) Representative: Schulz Junghans

(57) **Abstract**

The instant invention relates to compounds characterized by a general formula I and use thereof as a medicament, preferably for prevention or treatment of heart failure.

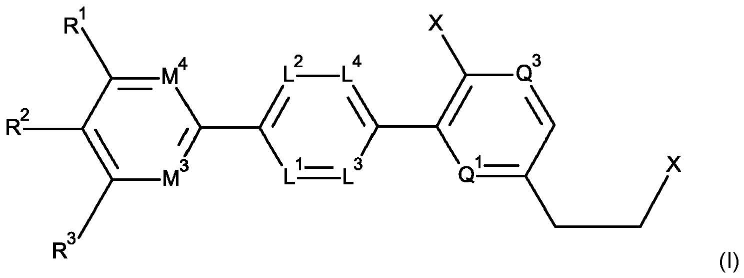

SEQUENCE LISTING

<110>  Max-Delbrueck Centrum fuer Molekulare Medizin

<120>  Substituted terpyridines

<130>  sca102ep-1

<160>  1

<170>  PatentIn version 3.5

<210>  1
<211>  25
<212>  PRT
<213>  Homo sapiens

<400>  1

Pro Glu Asp Ala Glu Leu Val Arg Leu Ser Lys Arg Leu Val Glu Asn
1               5                   10                  15

Ala Val Glu Lys Ala Val Gln Gln Tyr
            20                  25

## Description

The present invention relates to substituted terpyridines and use thereof as a medicament.

### Background

Protein kinase A (PKA) is a cAMP-dependent Ser/Thr kinase that plays a central role in cAMP-dependent signal transduction pathways. PKA consists of a dimer of regulatory (R) subunits, each of which binds one catalytic (C) subunit. The cAMP/PKA pathway controls a plethora of physiological processes. Therefore, a tight spatiotemporal control of PKA-activity is essential. One mechanism involved in the tight control of PKA activity is compartmentalization of PKA to distinct cellular sites by A-kinase anchoring proteins (AKAPs).

AKAP-PKA interactions have been shown to be functionally important in several pathological processes. The alpha and delta isoforms of AKAP18 play important roles in regulation of heart muscle contraction and water reabsorption in cells of the collection duct system of the kidney. Additionally, the inhibition of AKAP-PKA interactions has been recently suggested as a potential new therapeutic approach towards treatment of chronic heart failure. Non-peptidic, small molecules that inhibit the interaction between AKAPs and PKA therefore are promising drugs to target the PKA-AKAP interaction.

AKAPs are a family of around 50 scaffolding proteins. The defining characteristic of an AKAP is an amphipatic α-helix of 14 to 18 amino acids that is called an "R binding domain" (RBD) because it interacts with the R subunit dimer of PKA. More specifically, the RBDs of AKAPs bind to a domain of R subunits that is termed "Dimerization and Docking domain" (D/D domain). Most AKAPs preferentially interact with PKA RII type subunits, in which case the RBD can be more specifically referred to as an "RII-binding domain". RII binding domains are structurally conserved amphipathic α-helices which dock into the dimerization and docking domain of RII subunit dimers.

### Summary

Based on this background the objective of the present invention is to provide compounds, which can act as selective PKA-AKAP interaction inhibitors. The objective is attained by the subject-matter of the independent claim 1.

According to a first aspect, a compound characterized by a general formula I 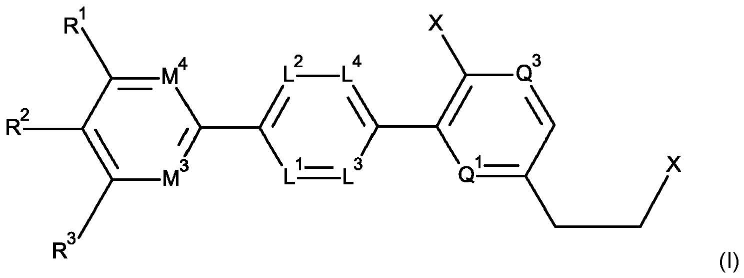 is provided, wherein
- R¹ is a moiety characterized by formula II 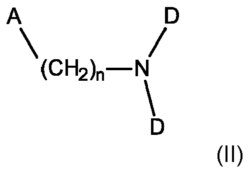 wherein
   - A is oxygen, CH₂, CHF, CHCl, CHBr, CHI, CF₂, CCl₂ or CFCl; n is 1, 2 or 3; each D independently is an aryl or heteroaryl, and the moiety is attached at A,
- R² is H or a moiety characterized by formula III 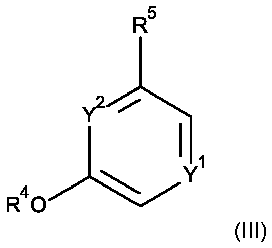 wherein
   - R⁴ is H or methyl, R⁵ is a C₂-C₄ alkyl, Y¹ and Y² can be CH or N, and the moiety is attached at the C atom in para-position to R⁵,
- R³ is
   - H or a C₁-C₄ alkyl and M³ is N or CH, or
   - R³ and M³ form a cyclic alkyl or alkylether moiety; for example a
      cyclopentyl 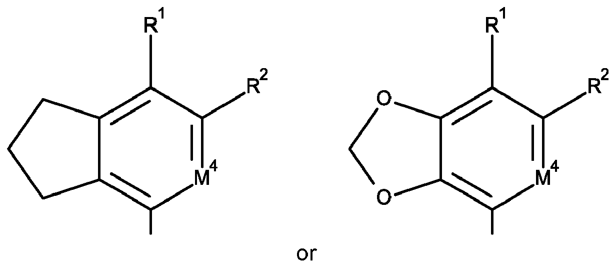 or 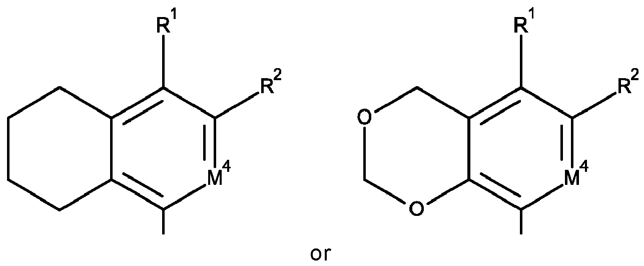
- M⁴ is N or CH;
- L¹ and L³ independently are CH, C-CH₃ or N; L² is N, CH or CH-COOH; and L⁴, Q¹ and Q³ independently are CH or N;
- each X independently can be a CN, COOR⁶, or CONR⁶₂, wherein each R⁶ independently can be H or C₁-C₄ alkyl;
with the proviso that
- if either of M³ or M⁴ are N, L¹ and L² are not N, and
- if L³ or L⁴ is N, Q¹ is CH.

The term aryl in the context of the present, signifies a cyclic aromatic C₅-C₁₀ hydrocarbon. Examples of aryl include, without being restricted to, cyclopentadiene, phenyl, benzyl (Bn) and naphthyl. A heteroaryl in the context of the present invention is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the invention additionally may be substituted by one or more alkyl groups.

The term alkyl or alkyl group in the context of the present invention signifies a saturated or partially unsaturated hydrocarbon moiety, which may be linear, branched, cyclic or cyclic with linear or branched side chains. The term alkyl includes partially unsaturated hydrocarbons such as propenyl, but not aromatic hydrocarbons. Examples are methyl, ethyl, n- or isobutyl. In another embodiment, the term alkyl may further encompass alkyl groups linked or bridged by hetero atoms such as N, S or O. Preferred alkyls are methyl, ethyl, propyl and butyl.

According to one embodiment, R¹ is moiety characterized by formula VI 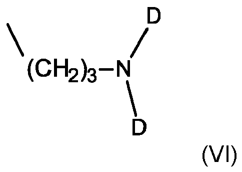 wherein each D has the meaning described in the preceding paragraphs.

According to one embodiment, D is cyclopentadien, phenyl, imidazole, pyridine, pyrimidine, benzyl (phenylmethylene), imidazolemethylene, pyridinemethylene or pyrimidinemethylene. Benzyl is preferred.

According to another embodiment, R² is 3-methoxy-5-propyl-pyridine: 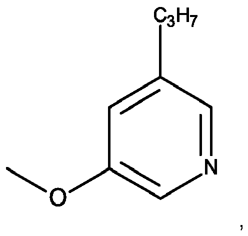 and is attached at position 2. Both n-propyl and iso-propyl moieties are considered.

According to yet another preferred embodiment, R³ is -CH₂-CH₂-CH₃ or R³ and M³ form a cyclopentyl moiety.

According to yet another preferred embodiment, X is CO₂H (or COO⁻).

According to a more preferred embodiment, L₃ is CH₂CO₂H, X is CO₂H and D is benzyl for any of the above embodiments.

According to another preferred embodiment, a compound characterized by a general formula IV 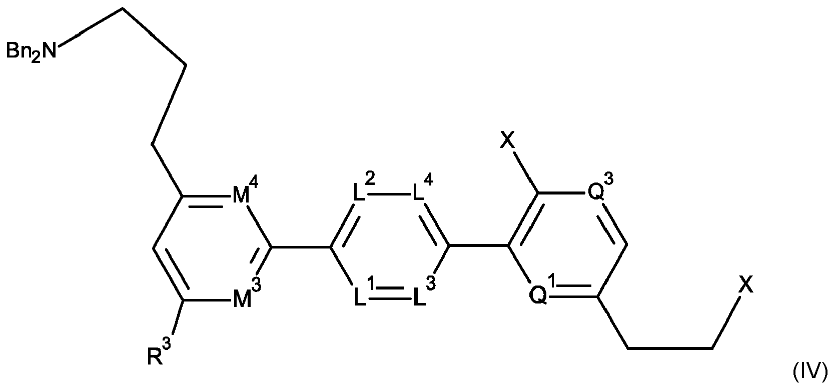 is provided, wherein
- R³ is
   - H or a C₁-C₄ alkyl and M³ is N or CH, or
   - R³ and M³ form a cyclic alkyl or alkylether moiety,
- M⁴ is N or CH,
- L¹ and L³ independently are CH, C-CH₃ or N; L² is N, CH or CH-COOH; and L⁴, Q¹ and Q³ independently are CH or N;
- each X independently is a CN, COOR⁶, or CONR⁶₂, wherein each R⁶ independently is H or C₁-C₄ alkyl,
with the proviso that
- if either of M³ or M⁴ are N, L¹ and L² are not N; and
- if L³ or L⁴ is N, Q¹ is CH.

According to another preferred embodiment, a compound characterized by a general formula V 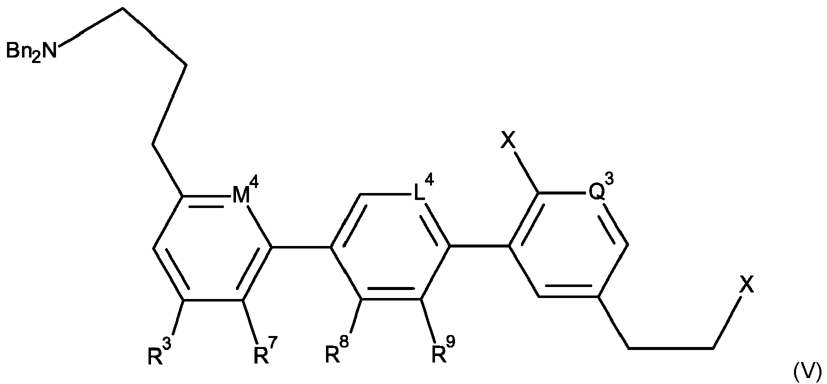 is provided, wherein
- M⁴, L⁴ and Q³ independently are CH or N,
- R³ and R⁷ independently are H or C₁-C₄-alkyl, or R³ and R⁷ form a cyclic alkyl or alkylether moiety,
- R⁸ and R⁹ independently are H or CH₃,
- each X independently can be a CN, COOR⁶, or CONR⁶₂, wherein each R⁶ independently can be H or C₁-C₄ alkyl.

According to a more preferred embodiment, a compound selected from the group comprised of:
- 3-[6-carbamoyl-5-[5-[3-[3-(dibenzylamino)propyl]-6,7-dihydro-5H-cyclopenta[c]pyridin-1-yl]-3,4-dimethyl-2-pyridyl]-3-pyridyl]propanoic acid: 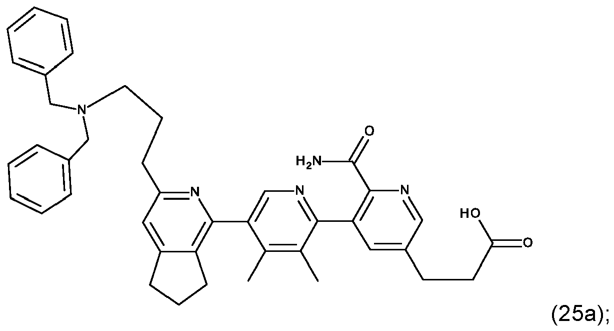
- 3-[6-carbamoyl-5-[5-[3-[3-(dibenzylamino)propyl]-6,7-dihydro-5H-cyclopenta[c]pyridin-1-yl]-3-methyl-2-pyridyl]-3-pyridyl]propanoic acid: 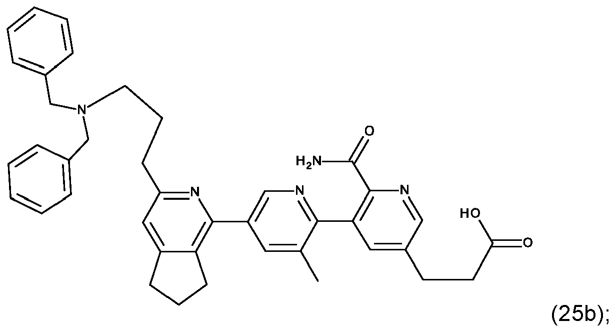
- alkyl 3-[6-cyano-5-[5-[3-[3-(dibenzylamino)propyl]-6,7-dihydro-5H-cyclopenta[c]pyridin-1-yl]-3,4-dimethyl-2-pyridyl]-3-pyridyl]propanoate: 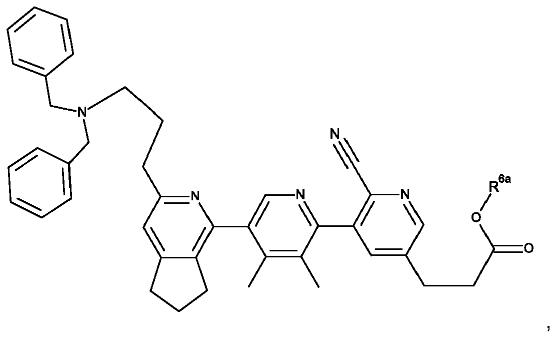 wherein
   R^{6a} is C₁-C₄-alkyl, preferably ethyl (22a);
- alkyl 3-[6-cyano-5-[5-[3-[3-(dibenzylamino)propyl]-6,7-dihydro-5H-cyclopenta[c]pyridin-1-yl]-3-methyl-2-pyridyl]-3-pyridyl]propanoate: 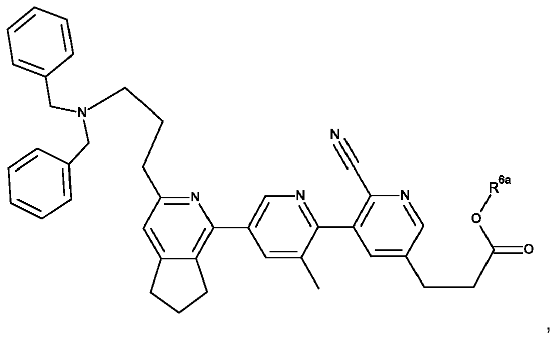 wherein
   R^{6a} is C₁-C₄-alkyl, preferably ethyl (22b);
- alkyl 3-[5-[5-[3-[1-chloro-3-(dibenzylamino)propyl]-6,7-dihydro-5H-cyclopenta[c]pyridin-1-yl]-3-methyl-2-pyridyl]-6-cyano-3-pyridyl]propanoate: 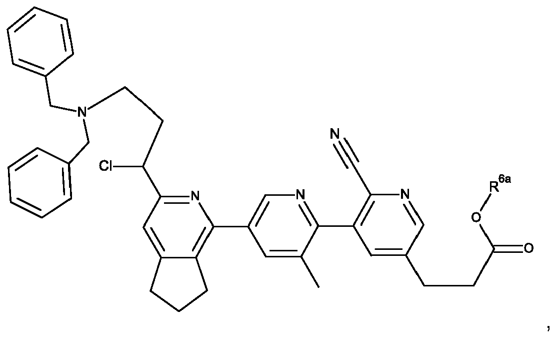 wherein
   R^{6a} is C₁-C₄-alkyl, preferably ethyl (22c);
- 3-[6-carbamoyl-5-[5-[4-(3-methoxy-5-propyl-2-pyridyl)-3-[2-(N-phenylanilino)ethoxy]phenyl]-3,4-dimethyl-2-pyridyl]-3-pyridyl]propanoic acid 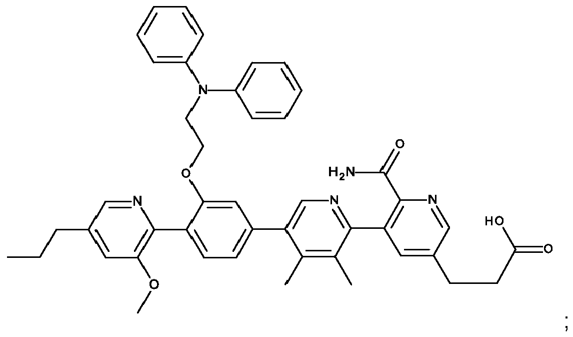
is provided.

According to another aspect of the invention, the following intermediates used in the synthesis of the compounds of the invention are provided:
- ethyl-3-[6-cyano-5-(4,4,5,5-tetramethyl-1,3,2-dioxaborolan-2-yl)pyridin-3-yl]propanoate (6f)
- 3-(6,7-dihydro-5H-cyclopenta[c]pyridin-3-yl)propanenitrile (8a)
- 3-(6,7-dihydro-5H-cyclopenta[c]pyridin-3-yl)propan-1-amine (8b)
- *N,N*-dibenzyl-3-(6,7-dihydro-5H-cyclopenta[c]pyridin-3-yl)propan-1-amine (8c)
- *N,N*-dibenzyl-3-(1-bromo-6,7-dihydro-5H-cyclopenta[c]pyridine-3-yl)propan-1-amine (8d)
- *N,N*-dibenzyl-3-(1-chloro-6,7-dihydro-5H-cyclopenta[c]pyridine-3-yl)propan-1-amine (8e)
- (rac)-*N,N*-dibenzyl-3-chloro-3-(1-chloro-6,7-dihydro-5H-cyclopenta[c]pyridine-3-yl)propan-1-amine (8I)
- ethyl 3-(5-bromo-2'-cyano-3,4-dimethyl-2,3'-bipyridin-5'-yl)propanoate (12a)
- ethyl 3-(5-bromo-2'-cyano-3-methyl-2,3'-bipyridin-5'-yl)propanoate (12b).

According to another aspect of the invention, a compound according to the above aspect of the invention is used as a medicament. Such medicament may be used for prevention or treatment of AKAP-PKA-interaction-associated diseases such as cardiovascular diseases, particularly chronic or acute heart failure or indications caused by excessive water retention such as syndrome of inappropriate antidiuretic hormone hypersecretion, cirrhosis caused by alcohol abuse or neoplastic diseases, or water retention during pregnancy.

According to yet another aspect of the invention, a pharmaceutical composition is provided, comprising a compound according to the above aspect of the invention. Pharmaceutical compositions for enteral administration, such as nasal, buccal, rectal or, especially, oral administration, and for parenteral administration, such as subcutaneous, intravenous, intrahepatic or intramuscular administration, are especially preferred. The pharmaceutical compositions comprise from approximately 1% to approximately 95% active ingredient, preferably from approximately 20% to approximately 90% active ingredient.

According to another aspect of the invention, a dosage form is provided, comprising a compound according to the above aspect of the invention. Dosage forms may be for enteral administration, such as nasal, buccal, rectal, transdermal or oral administration, or as an inhalation formulation or suppository. Alternatively, parenteral administration may be used, such as intravenous, intrahepatic, or especially subcutaneous, or intramuscular injection forms. Optionally, a pharmaceutical carrier or excipient may be present.

Such pharmaceutical composition or such dosage form described in the preceding paragraphs may be used in prevention or treatment of any AKAP-PKA-interaction associated disease. Preferably, a pharmaceutical composition or a dosage form according to the above aspects of the invention is used for prevention or treatment of cardiovascular diseases, more preferably of chronic or acute heart failure.

According to another aspect of the invention, a method for manufacture of a medicament is provided, comprising the use of a compound according to the above aspect of the invention. Medicaments according to the invention are manufactured by methods known in the art, especially by conventional mixing, coating, granulating, dissolving or lyophilizing.

According to yet another aspect of the invention, a method for prevention or treatment of cardiovascular diseases, particularly chronic or acute heart failure, is provided, comprising the administration of a compound according to the above aspect of the invention to a patient in need thereof.

The treatment may be for prophylactic or therapeutic purposes. For administration, a compound according to the above aspect of the invention is preferably provided in the form of a pharmaceutical preparation comprising the compound in chemically pure form and optionally a pharmaceutically acceptable carrier and optionally adjuvants. The compound is used in an amount effective against cardiovascular diseases such as chronic or acute heart failure. The dosage of the compound depends upon the species, the patient age, weight, and individual condition, the individual pharmacokinetic data, mode of administration, and whether the administration is for prophylactic or therapeutic purposes. The daily dose administered ranges from approximately 0.1 mg/kg to approximately 1000 mg/kg, preferably from approximately 0.5 mg to approximately 100 mg/kg, of the active agent according to the invention.

### Description of the figures

Fig. 1 shows ligand 2a (A) and a view of ligand 2a docked into the D/D domain of the RIIα-subunit of PKA (B).
Fig. 2 shows a schematic view of the proposed interaction of ligand 2a and its binding site within the D/D domain of the RIIα-subunit of PKA.
Fig. 3 shows the quarterpyridines ligands 2a and derivative 2b.
Fig. 4 shows the 1H-NMR spectra from STD measurements of RIIαIf-His with indicated compound, top: off-resonance spectrum, bottom: STD spectrum, left: 25b and right: 14b.
Fig. 5 shows HSQC spectra of D/D domain, black: ¹H-¹⁵N HSQC of 100 µM ¹⁵N-labeled D/D domain of RIIα, grey: ¹H-¹⁵N HSQC of 100 µM ¹⁵N-labeled D/D domain of RIIα incubated with 300µM of 25b.

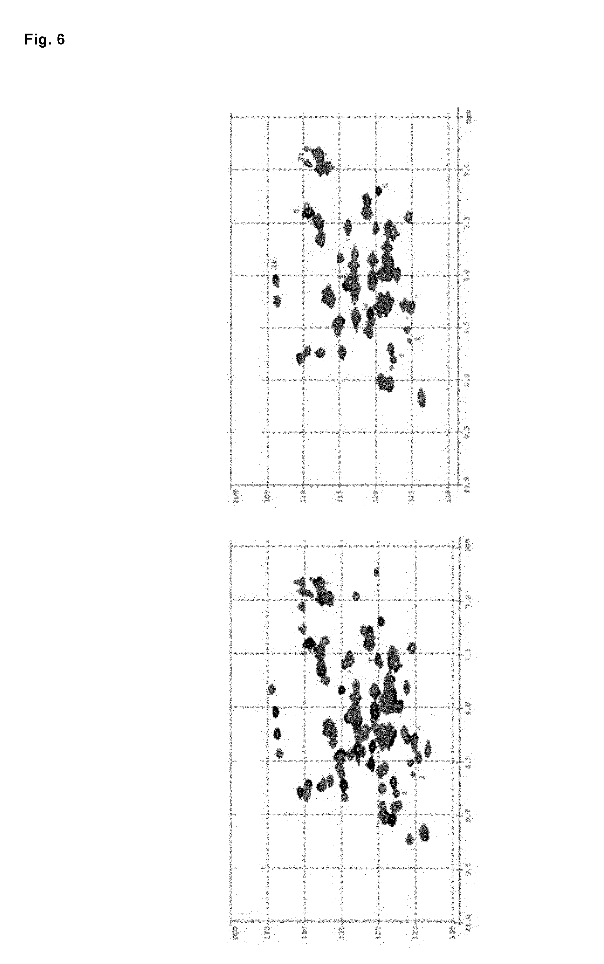

Fig. 6 shows HSQC spectra of D/D domain, black: ¹H-¹⁵N HSQC of 100 µM ¹⁵N-labeled D/D domain of RIIα, grey: ¹H-¹⁵N HSQC of 100 µM ¹⁵N-labeled D/D domain of RIIα incubated with 800µM of peptide, left: 16-mer AKAP18δL314E_6-21 and right:14-mer AKAP18δL314E_9-22.

### Detailed Description

### Example 1: Design of the quarterpyridine scaffold:

Peptides derived from the RII-binding domain (RIIBD) of AKAP18 bind with subnanomolar affinity (KD = 0.4 nM; SPR-measurements) to RII subunits of PKA. Thereby these peptides inhibit interaction of PKA with AKAPs. The peptide AKAP18δ-L314E (PEDAELVRLSKRLVENAVEKAVQQY (SEQ ID 01)) inhibits AKAP18α-PKA interaction with an IC₅₀ in the low nanomolar range.

For the development of drug-like mimics of RIIBD of the AKAP18 family, selected basic scaffolds were virtually docked using MOE software (Molecular Operating Environment Chemical Computing Group, Montreal, CA) into the proposed binding site of the peptide AKAP18δ-L314E within the D/D domain of RIIα; they were functionalized on the basis of the peptide AKAP18δ-L314E and ranked for binding potential.

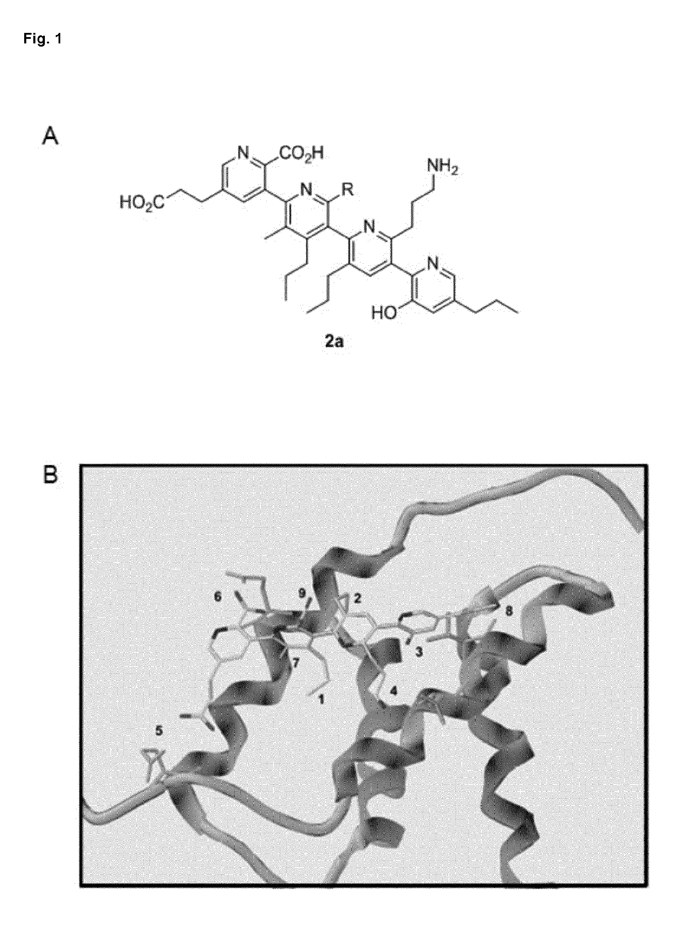

The pyridine-based oligomer **2a** (Fig. 1A) was designed *in silico* as a potential non-peptidic mimic of the RIIBD of AKAP18δ. Modelling was performed with MOE software (Molecular Operating Environment).

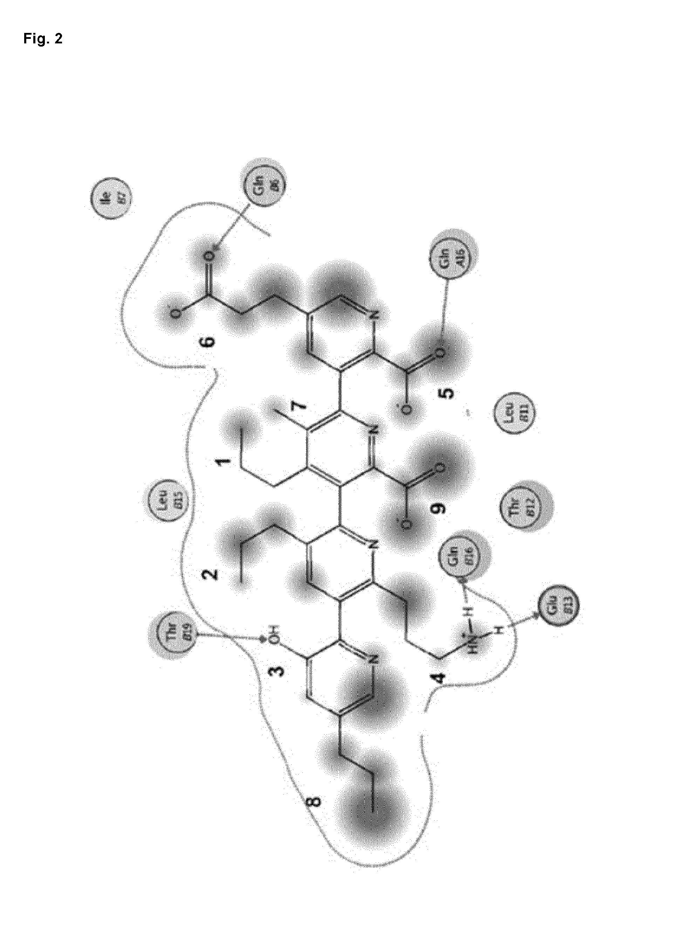

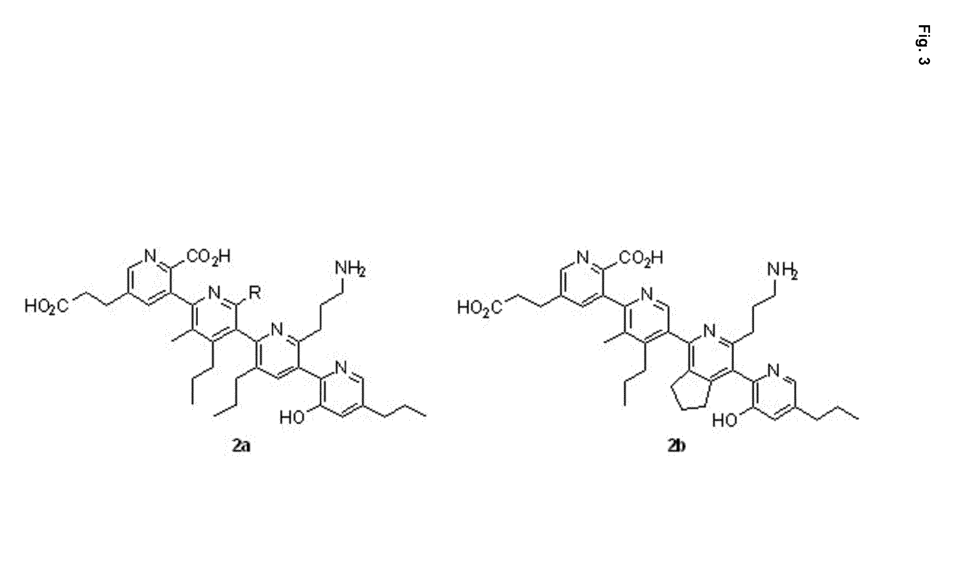

The best overlap between AKAP18δL314E and a potential mimic was found with a 2,3'-linked quaterpyridine as backbone scaffold, as can be seen in Fig. 1B and 2. Some modifications to quaterpyridine **2a** were introduced in order to simplify the synthesis, resulting in target structure **2b** which is depicted in Fig. 3.

The hydrophilic residue 9 that was originally introduced to increase water solubility of the final product was removed from the structure. No major solubility issues were anticipated for the final ligand due to the hydrophilic nature of the pyridine backbone.

In addition, residue 2 from Fig. 3 was replaced by a fused cyclopentyl group attached to C-3 and C-4 of the pyridine in order to simplify the synthesis and allow for easy modifications at this position later on.

### Example 2: Synthesis of terpyridines

### Ethyl-3-[6-cyano-5-(4,4,5,5-tetramethyl-1,3,2-dioxaborolan-2-yl)pyridin-3-yl]propanoate (6f)

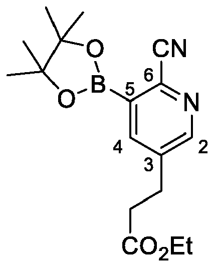

Into an oven-dried 100 ml three-necked flask equipped with magnetic stir bar, nitrogen inlet, rubber septum and bubbler was placed 2,2,6,6-tetramethylpiperidine (0.99 ml, 6.0 mmol) in anhydrous THF (10 ml) under nitrogen atmosphere. The reaction mixture was cooled down to 0°C, then 2.5 M n-butyllithium in hexane (2.35 ml, 6.0 mmol) was added dropwise into the flask via syringe and the reaction mixture was stirred for 30 minutes at 0°C. After cooling down to -75°C, ethyl 3-(6-cyanopyridin-3-yl)propanoate (CAS Nr. 917761-05-8, 0.3 g, 1.5 mmol) in THF was added dropwise via syringe. The reaction mixture turned deep brown and was stirred for 15 minutes at -75°C. Neat triisopropylborate (1.7 ml, 7.5 mmol) was added quickly to the reaction mixture, which was then stirred for 1 hour at -75°C. Then, pinacol (0.89 g, 7.7 mmol) in THF (2 ml) was added quickly to the reaction mixture. The solution was warmed up in a water bath (T = 40°C) and stirred for 2 hours. The reaction was quenched with water. The aqueous phase was acidified to pH 4 with 1M hydrochloric acid and extracted three times with chloroform. The combined organic phases were dried over MgSO₄ and the solvent was removed under reduced pressure. The product hydrolyzed to the free boronic acid under conditions used in HPLC and LC-MS runs. Analytical data represent results for the boronic acid.

The product was used without further purification in the next reaction step.
**HRMS:** cal. 249.1043, exp. 249.1032 ([M+H]⁺)
**ESI-MS:** cal. 249.1043, exp. 249.1 ([M+H]⁺)
***t*_{R}(HPLC)** 4.18 min

### 3-(6,7-dihydro-5H-cyclopenta[c]pyridin-3-yl)propanenitrile (8a)

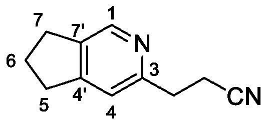

An oven-dried 500 ml three-necked flask equipped with magnetic stir bar, nitrogen inlet and bubbler was charged under nitrogen atmosphere with succinonitrile (10 g, 125 mmol) and Cp*Ru(cod)Cl (600 mg, 1.63 mmol) in 1,2-dichloroethane (300 ml). The reaction mixture was cooled down to 0°C and 1,6-heptadiyne (7.6 g, 82.5 mmol) in 1,2-dichloroethane (50 ml) was added slowly via dropping funnel over 30 minutes. The reaction mixture was warmed up to room temperature and stirred for 20 hours, and then the mixture was concentrated *in vacuo*. Column chromatography of the residue (EtOAc:hexane 2+1) gave the product as white solid in 79% yield (11.2 g).

**¹H-NMR** (300 MHz, CDCl₃): δ 2.06-2.16 (m, 2H, H-6), 2.80-2.85 (t, ³*J*= 7.3, 2H, -C**H₂**-CN), 2.89-2.94 (m, 4H, H-5, H-7), 3.05-3.10 (t, ³*J* = 7.3, 2H, -C**H₂**-CH₂-CN), 7.11 (s, 1H, H-4), 8.38 (s, 1H, H-1) **¹³C-NMR** (75,5 MHz, CDCl₃): δ 17.32 (-**C**H₂-CN), 25.24 (C-6), 30.14, 32.84 (C-5, C-7), 33.44 (-**C**H₂-CH₂-CN), 119.64 (C-4), 119.74 (-**C**N), 139.12 (C-7'), 145.27 (C-1), 154.71, 155.35 (C-3, C-4').
**HRMS:** cal. 173.1073, exp. 173.1070 ([M+H]⁺)
**ESI-MS:** cal. 173.1073, exp. 173.0 ([M+H]⁺)

| | | | |
|---|---|---|---|
| **m.p.** | 68.5°C | | |
| **R_{f}(TLC)** | 0.29 (EtOAc:hexane 2+1) | ***t*_{R}(HPLC)** | 1.13 min |

### 3-(6,7-dihydro-5H-cyclopenta[c]pyridin-3-yl)propan-1-amine (8b)

An oven-dried 500 ml three-necked flask equipped with magnetic stir bar, nitrogen inlet, condenser with bubbler and dropping funnel was charged with **8a** (5 g, 29 mmol) in anhydrous THF (200 ml) under nitrogen atmosphere. Neat BH₃*SMe₂ (12.6 g, 165.3 mmol) was added in one portion to the solution via dropping funnel. The reaction mixture was heated up to 60°C and stirred at this temperature over night. The solution was cooled down to room temperature, and then nitrogen inlet and dropping funnel were removed from the apparatus. 10 M hydrochloric acid was added very carefully (gas development!) until the white precipitate was completely dissolved. The reaction mixture was then heated to 70°C and stirred for 3 hours. After cooling down to room temperature, the aqueous phase was washed three times with diethyl ether. The aqueous phase was cooled down to 0°C and treated with 10 M sodium hydroxide solution to pH >11. The aqueous phase was extracted three times with chloroform. The combined organic phases were dried over a 1:1 mixture of MgSO₄ and K₂CO₃. Removal of the solvent under reduced pressure gave the crude product as light brown oil in 86% yield (4.4 g). The product was used without further purification in the next reaction step.

**¹H-NMR** (300 MHz, d₆-DMSO): δ 1.65-1.75 (m, 2H, -C**H₂**-CH₂-NH₂), 1.95-2.05 (m, 2H, H-6), 2.51-2.56 (t, ³*J* = 7.0, 2H, -C**H₂**-NH₂), 2.66-2.71 (t, ³*J* = 7.6, 2H, -C**H₂**-CH₂-CH₂-NH₂), 2.80-2.85 (m, 4H, H-5, H-7), 7.11 (s, 1H, H-4), 8.29 (s, 1H, H-1).

**¹³C-NMR (75,5** MHz, CDCl₃): δ 25.27, 30.10 (C-6, -CH₂-CH₂-NH₂), 31.35, 32.83 (C-5, C-7), 35.64 (-CH₂-CH₂-CH₂-NH₂), 41.26 (-CH₂-CH₂-CH₂-NH₂), 119.40 (C-4), 138.05 (C-7'), 144.70 (C-1), 155.12, 158.54 (C-3, C-4').
**HRMS:** cal. 177.1386, exp. 177.1380 ([M+H]⁺)
**ESI-MS:** cal. 177.1386, exp. 177.1 ([M+H]⁺)
***t*_{R}(HPLC)** 1.04 min

### N,N-dibenzyl-3-(6,7-dihydro-5H-cyclopenta[c]pyridin-3-yl)propan-1-amine (8c)

Into an one-necked 100 ml flask was placed **8b** (1.4 g, 8 mmol), benzyl bromide (1.9 ml,16 mmol) and triethylamine (2.23 ml, 16 mmol) in acetonitrile (75 ml). The reaction mixture was stirred for 3 hours at 60°C or for 24 hours at room temperature. The solvent was removed under reduced pressure and the residue was partitioned between brine and chloroform. The aqueous phase was extracted twice with chloroform. The combined organic phases were dried over MgSO₄; the solvent was removed under reduced pressure. Column chromatography (EtOAc/hexane 1:2 containing 2% NEt₃) gave the product as yellow oil in 51 % yield (1.45 g).

**¹H-NMR** (300 MHz, d₆-DMSO): δ 1.80-1.90 (m, 2H,-CH₂-CH₂-NBn₂), 1.93-2.03 (m, 2H, H-6), 2.35-2.40 (t, ³*J* = 7.0, 2H, -C**H₂**-NBn₂), 2.58-2.63 (t, ³*J* = 7.5, 2H, -C**H₂**-CH₂-CH₂-NBn₂), 2.75-2.83 (m, 4H, H-5, H-7), 3.52 (s, 4H, Bn-H), 6.92 (s, 1H, H-4), 7.22-7.32 (m, 10H, Bn-H ), 8.26 (s,1H, H-1).

**¹³C-NMR** (75,5 MHz, d₆-DMSO): δ 24.62 (C-6), 26.51 (-CH₂-CH₂-NBn₂), 29.25 (C-7), 31.92 (C-5), 34.94 (-**C**H₂-CH₂-CH₂-NBn₂), 52.12 (-**C**H₂-NBn₂), 57.48 (Bn-C), 118.52 (C-4), 126.69 (Bn-C), 128.09 (Bn-C), 128.46 (Bn-C),136.79 (Bn-C), 139.50 (C-7'), 144.36 (C-1), 153.35 (C-4'), 158.72 (C-3).

| | | | |
|---|---|---|---|
| **HRMS:** | cal. 357.2325 exp. 357.2329 ([M+H]⁺) | | |
| **ESI-MS:** | cal. 357.2325, exp. 357.2 ([M+H]⁺) | | |
| **R_{f}(TLC)** | 0.53 (EtOAc:hexane = 1:2 with 2% NEt₃) | ***t*_{R}(HPLC)** | 2.86 min |

### N,N-dibenzyl-3-(1-bromo-6,7-dihydro-5H-cyclopenta[c]pyridine-3-yl)propan-1-amine (8d)

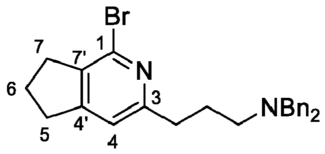

An oven-dried 500 ml three-necked flask equipped with magnetic stir bar, nitrogen inlet, bubbler and rubber septum was charged with 2-dimetyhlaminoethanol (2.3 ml, 22.5 mmol) in anhydrous hexane under nitrogen atmosphere. The reaction mixture was cooled down to 0°C and 2.5M nBuLi in hexane (17.9 ml, 44.9 mmol) was added to the solution by syringe. The mixture was stirred for 15 minutes at 0°C and then cooled down to -65°C. **8c** (2 g, 5.6 mmol) in diethyl ether was added to the reaction mixture by syringe. The solution turned deep red and was stirred at -65°C for 30 minutes. The mixture was cooled down to -75°C and tetrabromomethane (11.1 g, 42 mmol) in hexane (15 ml) was added quickly via dropping funnel. The reaction mixture was stirred at -75°C for 30 minutes and quenched with water. The aqueous phase was extracted three times with chloroform. The combined organic phases were dried over MgSO₄ and the solvent was removed under reduced pressure. Column chromatography (EtOAc/hexane containing 1 % NEt₃: gradient starting with 0% EtOAc, product eluted at 10% EtOAc) gave the product as yellow oil in 36% yield (878 mg).

**¹H-NMR** (300 MHz, d₆-DMSO): δ 1.83-1.88 (m, 2H, -C**H₂**-CH₂-NBn₂), 1.96-2.07 (m, 2H, H-6), 2.34-2.39 (t, ³*J* = 6.9, 2H, -C**H₂**-NBn₂), 2.58-2.62 (t, ³*J* = 7.4, 2H,-C**H₂**-CH₂-CH₂-NBn₂), 2.77-2.82 (t, ³*J* = 7.5, 2H, H-7), 2.87-2.92 (t, ³*J* = 7.6, 2H, H-5), 3.52 (s, 4H, Bn-H), 6.95 (s, 1H, H-4), 7.22-7.32 (m, 10H, Bn-H).

**¹³C-NMR** (75,5 MHz, d₆-DMSO): δ 23.11 (C-6), 26.11 (-CH₂-CH₂-NBn₂), 32.11 (C-7), 33.14 (C-5), 34.22 (-CH₂-CH₂-CH₂-NBn₂), 51.77 (-**C**H₂-NBn₂), 57.48 (Bn-C), 118.66 (C-4), 126.70 (Bn-C), 128.09 (Bn-C), 128.47 (Bn-C),137.59 (C-7'), 138.25 (C-1), 139.43 (Bn-C), 156.49 (C-4'), 160.79 (C-3).

| | | | |
|---|---|---|---|
| **HRMS:** | cal. 435.1430, exp. 435.1428 ([M+H]⁺) | | |
| **ESI-MS:** | cal. 435.1430, exp. 435.1 ([M+H]⁺) | | |
| **m.p.** | 63.9°C | | |
| **R_{f}(TLC)** | 0.58 (EtOAc:hexane = 1:6 with 1% NEt₃) | ***t*_{R}(HPLC)** | 5.56 min |

### N,N-dibenzyl-3-(1-chloro-6,7-dihydro-5H-cyclopenta[c]pyridine-3-yl)propan-1-amine (8e)

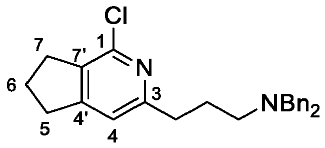

An oven-dried 500 ml three-necked flask equipped with magnetic stir bar, nitrogen inlet, bubbler and dropping funnel was charged with 2-dimetyhlaminoethanol (5.8 ml, 56 mmol) in anhydrous hexane under nitrogen atmosphere. The reaction mixture was cooled down to 0°C and 2.5M nBuLi in hexane (44.8 ml, 112 mmol) was added to the solution by syringe. The mixture was stirred for 15 minutes at 0°C and then cooled down to -65°C. **8c** (5 g, 14 mmol) in diethyl ether was added to reaction mixture by syringe. The solution turned deep red and was stirred at -65°C for 30 minutes. The mixture was cooled down to -75°C and hexachloroethane (16.5 g, 70 mmol) in hexane (45 ml) was added in one shot by dropping funnel. The reaction mixture was stirred at -75°C for 30 minutes and quenched with water. The aqueous phase was extracted three times with chloroform. The combined organic phases were dried over MgSO₄ and the solvent was removed under reduced pressure. Column chromatography (EtOAc/hexane containing 1 % NEt₃: gradient starting with 0% EtOAc, product eluted at 10% EtOAc) gave the product as white solid in 59% yield (3.2 g).

**¹H-NMR** (300 MHz, d₆-DMSO): δ 1.79-1.89 (m, 2H, -C**H₂**-CH₂-NBn₂), 1.98-2.08 (m, 2H, H-6), 2.34-2.39 (t, ³*J* = 7.0, 2H, -C**H₂**-NBn₂), 2.57-2.62 (t, ³*J* = 7.4, 2H, -C**H₂**-CH₂-CH₂-NBn₂), 2.80-2.90 (m, 4H, H-5, H-7), 3.52 (s, 4H, Bn-H), 6.93 (s, 1H, H-4), 7.21-7.30 (m, 10H, Bn-H). **¹³C-NMR** (75,5 MHz, d₆-DMSO): δ 23.46 (C-6), 26.08 (-**C**H₂-CH₂-NBn₂), 30.38 (C-7), 32.86 (C-5), 34.27 (-CH₂-CH₂-CH₂-NBn₂), 51.78 (-**C**H₂-NBn₂), 57.48 (Bn-C), 118.38 (C-4), 126.70 (Bn-C), 128.08 (Bn-C), 128.48 (Bn-C),135.27 (C-7'), 139.43 (Bn-C), 145.46 (C-1), 157.24 (C4'), 160.34 (C-3).

| | | | |
|---|---|---|---|
| **HRMS:** | cal. 391.1936 exp. 391.1930 ([M+H]⁺) | | |
| **ESI-MS:** | cal. 391.1936, exp. 391.2 ([M+H]⁺) | | |
| **m.p.** | 48.7°C | | |
| **R_{f}(TLC)** | 0.56 (EtOAc:hexane = 1:6 with 1% NEt₃) | ***t*_{R}(HPLC)** | 7.34 min |

### (rac)-N,N-dibenzyl-3-chloro-3-(1-chloro-6,7-dihydro-5H-cyclopenta[c]pyridine-3-yl)propan-1-amine (8I)

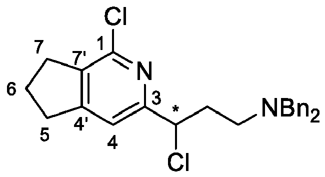

An oven-dried 500 ml three-necked flask equipped with magnetic stir bar, nitrogen inlet, bubbler and dropping funnel was charged with potassium *t*-butoxide (2.3 g, 20.5 mmol) under nitrogen atmosphere. Anhydrous diethyl ether (250 ml) was added, the solution was cooled down to -75°C and 2.5 M nBuLi in hexane (8.2 ml, 20.5 mmol) was added to the solution by syringe. The mixture was stirred for 10 minutes at -75°C. **8e** (4 g, 10.3 mmol) in diethyl ether was added to the reaction mixture by dropping funnel. The solution turned deep red and was stirred at -75°C for 60 minutes. Hexachloroethane (12.1 g, 51.3 mmol) in hexane (30 ml) was added quickly by dropping funnel. The reaction mixture was stirred at -75°C for 60 minutes and quenched with water. The aqueous phase was extracted three times with chloroform. The combined organic phases were dried over MgSO₄ and the solvent was removed under reduced pressure. Column chromatography (EtOAc/hexane containing 1% NEt₃: gradient starting with 0% EtOAc, product eluted at 10% EtOAc) gave the product as yellow oil in 33% yield (1.4 g).

**¹H-NMR** (300 MHz, CDCl₃): δ 2.06-2.16 (m, 2H, H-6), 2.27-2.42 (m, 2H, -C**H₂**-CH₂-NBn₂), 2.55-2.59 (t, ³*J* = 6.5, 2H, -C**H₂**-NBn₂), 2.90-2.95 (m, 4H, H-5, H-7), 3.56 (s, 4H, Bn-H), 4.96-5.01 (t, ³*J* = 7.0, 1H, -C**H**(Cl)-), 7.04 (s, 1H, H-4), 7.21-7.33 (m,10H, Bn-H).

**¹³C-NMR** (75,5 MHz, CDCl₃): δ 24.07 (C-6), 31.29, 33.72 (C-5, C-7), 35.77 (-**C**H₂-CH₂-NBn₂), 50.51 (-**C**H₂-NBn₂), 58.56 (Bn-C), 61.17 (-**C**H(Cl)-), 117.45 (C-4), 127.04 (Bn-C), 128.36 (Bn-C), 129.08 (Bn-C),138.51 (Bn-C), 139.54 (C-7'), 146.89 (C-1), 157.79 (C-3), 158.62 (C-4').

| | | | |
|---|---|---|---|
| **HRMS:** | cal. 425.1546, exp. 425.1540 ([M+H]⁺) | | |
| **ESI-MS:** | cal. 425.1546, exp. 425.2 ([M+H]⁺) | | |
| **R_{f}(TLC)** | 0.65 (EtOAc:hexane = 1:6 with 1% NEt₃) | ***t*_{R}(HPLC)** | 6.67 min |

### Ethyl 3-(5-bromo-2'-cyano-3,4-dimethyl-2,3'-bipyridin-5'-yl)propanoate (12a)

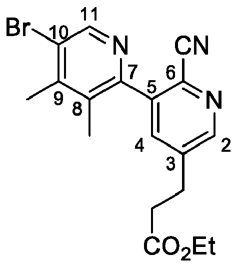

An oven-dried 250 ml three-necked flask equipped with magnetic stir bar, condenser with bubbler, nitrogen inlet and dropping funnel was charged with **6f** (4.1 g, 12.7 mmol), 2,5-dibromo-3,4-dimethylpyridine (CAS Nr. 125419-92-3, 2.0 g, 7.5 mmol), palladium (II) acetate (0.1 g, 0.45 mmol) and triphenylphosphine (0.5 g, 1.8 mmol). After evacuation and subsequent purging with N₂ for three times, dioxane (120 ml) and 1M potassium phosphate (5.7 ml) in H₂O were added by dropping funnel to the solution. The reaction mixture was heated to 80°C and stirred for 4 hours at this temperature. After cooling down to room temperature, the reaction mixture was diluted with brine and extracted three times with ethyl acetate. The combined organic phases were dried over MgSO₄ and the solvent was removed under reduced pressure. Column chromatography (EtOAc/hexane 1:4 containing 1 % NEt₃) gave the product in 56% yield as yellow viscous liquid (1.6 g).

**¹H-NMR** (300 MHz, d₆-DMSO): δ 1.11-1.16 (t, ³*J* = 7.1, 3H, -CH₂-C**H₃**), 2.19 (s, 3H, -C**H₃**), 2.48 (s, 3H, -C**H₃**), 2.74-2.79 (t, ³*J* = 7.4, 2H, -C**H₂**-CO₂Et)), 3.00-3.05 (t, ³*J* = 7.4, 2H, -C**H₂**-CH₂-CO₂Et)), 4.00-4.07 (quartet, ³*J* = 7.1, 2H, -C**H₂**-CH₃), 7.98-7.99 (d, ⁴*J* = 1.9, 1H, H-4), 8.69 (s, 1H, H-11), 8.74-8.74 (d, ⁴*J* = 1.9, 1H, H-2)

**¹³C-NMR** (75,5 MHz, d₆-DMSO): δ 14.02 (-**C**H₂-CH₃), 16.81 (-**C**H₃), 19.35 (-**C**H₃), 27.25 (-**C**H₂-CH₂-CO₂Et), 33.56 (-**C**H₂-CO₂Et)), 60.00 (-**C**H₂-CH₃), 116.60 (-**C**N), 123.79 (C-10), 129.61 (C-6) 133.18 (C-8), 137.91 (C-4), 139.76 (C-5), 140.56 (C-3), 146.71 (C-9), 148.09 (C-11), 150.92 (C-2), 151.65 (C-7), 171.74 (-**C**O₂Et).

| | | | |
|---|---|---|---|
| **HRMS: :** | cal. 388.0655, 390.0637; exp. 388.0638, 390,0620 ([M+H]⁺) | | |
| **ESI-MS:** | cal. 388.0655; exp. 388.2 ([M+H]⁺) | | |
| **R_{f}(TLC)** | 0.24 (EtOAc:hexane 1:2) | ***t*_{R}(HPLC)** | 9.07 min |

### Ethyl 3-(5-bromo-2'-cyano-3-methyl-2,3'-bipyridin-5'-yl)propanoate (12b)

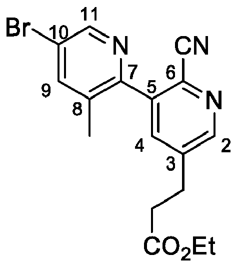

An oven-dried 250 ml three-necked flask equipped with magnetic stir bar, condenser with bubbler, nitrogen inlet and dropping funnel was charged with 2,5-dibromo-3-dimethylpyridine (2.2 g, 8.8 mmol), potassium phosphate (5.6 g, 26 mmol), palladium(II) acetate (0.46 g, 0.43 mmol) and triphenylphosphine (0.22 g, 1.7 mmol). After evacuation and subsequent purging with N₂ for three times, dioxane (45 ml) and H₂O (5 ml) were added via dropping funnel and the reaction mixture was stirred for 10 minutes at room temperature. After heating the mixture to 80°C, crude **6f** (6.6 g, about 8.8 mmol) in dioxane (15 ml) was added via dropping funnel over 90 minutes. The reaction mixture was stirred for 150 minutes at 80°C, cooled down to room temperature and stirred over night. The reaction mixture was diluted with brine and extracted three times with ethyl acetate. The combined organic phases were dried over MgSO₄ and the solvent was removed under reduced pressure. Column chromatography (EtOAc/hexane 1:4) gave the product in 74% yield (2.4 g) as yellow solid.

**¹H-NMR** (300 MHz, CDCl₃): δ 1.21-1.25 (t, ³*J* = 7.17, 3H, -CH₂-C**H₃**), 2.28 (s, 3H, -C**H₃**), 2.68-2.73 (t, ³*J* = 7.3, 2H, -C**H₂**-CO₂Et), 3.06-3.11 (t, ³*J* = 7.3, 2H, -C**H₂**-CH₂-CO₂Et), 4.09-4.16 (quartet, ³*J* = 7.14, 2H, -C**H₂**-CH₃), 7.70 (d, ⁴*J* = 1.9, 1H, H-4), 7.85 (d, ⁴*J* = 1.7, 1H, H-9), 8.64 (m, 2H, H-2, H-11).

**¹³C-NMR** (75,5 MHz, CDCl₃): δ 14.37 (-CH₂-**C**H₃), 19.14 (-**C**H₃), 28.19 (-**C**H₂-CH₂-CO₂Et), 34.75 (-CH₂-CO₂Et), 61.15 (-**C**H₂-CH₃), 116.47 (-**C**N), 121.53 (C-10), 131.31 (C-6), 134.11 (C-8), 137.80 (C-4), 139.93 (C-5), 140.21 (C-3), 141.33 (C-9), 148.85 (C-11), 151.02 (C-2), 151.69 (C-7), 171.89(-CO₂Et).

| | | | |
|---|---|---|---|
| **HRMS:** | cal. 374.0499; exp. 374.0496 ([M+H]⁺) | | |
| **ESI-MS:** | cal. 374.0499; exp. 374.1 ([M+H]⁺) | | |
| **m.p.** | 45.3°C | | |
| **R_{f}(TLC)** | 0.27 (EtOAc:hexane 1:2) | ***t*_{R}(HPLC)** | 7.52 min |

### Ethyl 3-(6-cyano-5-(5-(3-(3-(dibenzylamino)propyl)-6,7-dihydro-5H-cyclopenta[c]pyridin-1-yl)-3,4-dimethylpyridin-2-yl)pyridine-3-yl)propanoate (22a)

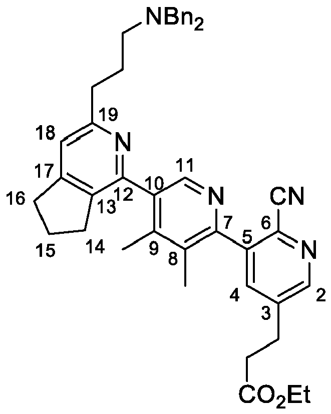

An oven-dried 5 ml microwave-flask equipped with magnetic stir bar was charged with **12a** (0.1 g, 0.26 mmol), potassium acetate (0.08 g, 0.39 mmol), bis(pinacolato)diboron (CAS Nr. 73183-34-3, 0.08 g, 0.31 mmol), tris(dibenzylideneacetone) dipalladium(0) (0.006 g, 0.006 mmol) and XPhos (0.025 g, 0.05 mmol) and was sealed with a microwave septum. The flask was evacuated and flushed with nitrogen. After addition of dioxane (2 ml), the reaction mixture was heated in the microwave to 130°C and stirred at this temperature for 15 minutes. After cooling down to room temperature, **8e** (*N,N*-dibenzyl-3-(1-chloro-6,7-dihydro-5*H-*cyclopenta[c]pyridine-3-yl)propan-1-amine, 0.08 g, 0.23 mmol) in dioxane (0.8 ml) and potassium phosphate (0.08 g, 0.39 mmol) in H₂O (0.3 ml) were added to reaction mixture via syringe. The reaction mixture was heated in the microwave to 130°C and stirred at this temperature for 30 minutes. After cooling down to room temperature, the reaction mixture was diluted with brine and extracted three times with ethyl acetate. The combined organic phases were dried over MgSO₄ and the solvent was removed under reduced pressure. Column chromatography (EtOAc/hexane 1:1 containing 1 % NEt₃) gave the product in 40% yield (69 mg) as orange oil.

The product was further purified by preparative HPLC (ACN/0.1 % TFA, H₂O/0.1% TFA) with the following gradient: 30% ACN to 99% ACN in H₂O in 50 minutes. **22a** eluted at t_{R} = 25.6 minutes and was obtained after lyophilization as violet ammonium trifluoroacetate salt.

**¹H-NMR** (300 MHz, MeOD): 1.16-1.21 (t, ³*J* = 7.1, 3H, -CH₂-C**H₃**), 2.10-2.30 (m, 10H, -C**H₂**-CH₂-NBn₂, -CH₃, H-15), 2.74-2.82 (m, 4H, -C**H₂**-CO₂Et, H-14), 2.87-2.92 (t, ³*J* = 7.3, 2H, H-16), 3.09-3.17 (m, 6H, -C**H₂**-CH₂-CH₂-NBn₂, -C**H₂**-NBn₂, -C**H₂**-CH₂-CO₂Et, H-14), 4.05-4.12 (quartet, ³*J* = 7.1, 2H, -C**H₂**-CH₃), 4.36 (s, 4H, Bn-H), 7.44 (m,10H, Bn-H), 7.50 (s, 1H, H-18), 7.95 (s, 1H, H-4), 8.37 (s, 1H, H-11), 8.71 (s, 1H, H-2).
**¹³C-NMR** (75,5 MHz, MeOD): δ 14.66 (-CH₂-**C**H₃), 16.52 (-**C**H₃), 17.35 (-**C**H₃), 24.64 (C-15), 26.04 (-CH₂-CH₂-NBn₂), 29.01 (-**C**H₂-CH₂-CO₂Et), 31.63 (C-14), 33.42 (C-16), 34.70 (-**C**H₂-CH₂-CH₂-NBn₂), 35.42 (-CH₂-CO₂Et), 52.32 (-**C**H₂-NBn₂), 58.56 (Bn-C), 61.93 (-**C**H₂-CH₃), 117.49 (-CN), 122.10 (C-18), 130.70 (Bn-C), 130.76 (Bn-C), 131.52 (Bn-C), 131.90 (C-6), 132.44 (Bn-C),134.18 (C-8), 136.28 (C-10 or C-1 3), 139.62 (C-4), 141.61, 141.74 (C-5, C-10 orC-13), 142.74 (C-3), 147.53 (C-11), 148.62 (C-9), 149.86 (C-12), 152.60 (C-2), 155.08 (C-7), 157.84 (C-17), 163.16 (C-19), 173.88 (-CO₂Et).

**HRMS:** cal. 664.3646, exp. 664.3643 ([M+H]⁺)
**ESI-MS:** cal. 664.3646, exp. 664.3 ([M+H]⁺)
**m.p.** 44.1°C
**R_{f}(TLC)** 0.38 (EtOAc:hexane 1:1 with 1% of NEt₃) ***t*_{R}(HPLC)** 6.04 min

### Ethyl 3-(2'-cyano-5-(3-(3-(dibenzylamino)propyl)-6,7-dihydro-5H-cyclopen ta[c]pyridine-1-yl)-3-methyl-2,3'-bipyridin-5'-yl)propanoate (22b)

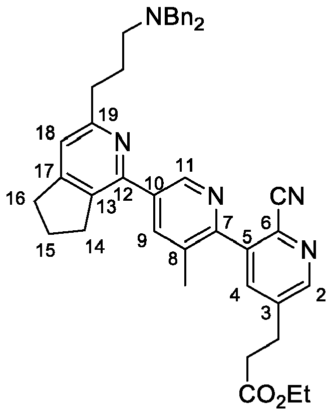

### Procedure A: microwave:

An oven-dried 20 ml microwave-flask equipped with magnetic stir bar was charged with **12b** (0.40 g, 1.1 mmol), potassium acetate (0.32 g, 3.2 mmol), bis(pinacolato)diboron (0.33 g, 1.3 mmol), the indicated amount of Pd(0) source and phosphine ligand and was sealed with a microwave septum. The flask was evacuated and flushed with nitrogen. After addition of 12 ml of the indicated solvent, the reaction mixture was heated in the microwave to 130°C and stirred at this temperature for 7 minutes (DMF) or 32 minutes (dioxane).

After cooling down to room temperature, dihydro-5*H*-cyclopenta[*c*]pyridine halide **8d** (0.75 mmol) or **8e** (0.75 mmol) in 3 ml of solvent and 1.6 mmol of base in 1 ml of H₂O were added to reaction mixture via syringe. The reaction mixture was heated in the microwave to 130°C and stirred at this temperature for 30-40 minutes. After cooling down to room temperature, the reaction mixture was diluted with brine and extracted three times with ethyl acetate. The combined organic phases were dried over MgSO₄ and the solvent was removed under reduced pressure. Column chromatography (EtOAc/hexane 1:1 containing 1% NEt₃) gave the product as orange oil.

| | **8d** | **8e** |
|---|---|---|
| Pd(0) source | Pd(OAc)₂ (12 mg, 0.05 mmol) | Pd₂dba₃ (25mg, 0.03 mmol) |
| phosphine | PPh₃ (56 mg, 0.21 mmol) | XPhos (102 mg, 0.2 mmol) |
| solvent | DMF | dioxane |
| base | K₂CO₃ (0.22 g, 1.6 mmol) | K₃PO₄ (0.34 g, 1.6 mmol) |
| yield | 48% (234 mg) | 54% (263 mg) |

### Procedure B: thermal:

An oven-dried 100 ml three-necked flask equipped with magnetic stir bar, nitrogen inlet, rubber septum and condenser with bubbler was charged with **12b** (0.15 g, 0.4 mmol), potassium acetate (0.12 g, 1.2 mmol), bis(pinacolato)diboron (0.12 g, 0.48 mmol),, tris(dibenzylideneacetone) dipalladium(0) (0.01 g, 0.01 mmol) and XPhos (0.04 g, 0.08 mmol). The flask was evacuated and flushed with nitrogen for at least three times. After addition of dioxane (3 ml) the reaction mixture was heated to 102°C and stirred at this temperature for 1 hour. Then, **8e** (0.1 g, 0.28 mmol) in dioxane (3ml) and potassium phosphate (0.13 g, 0.6 mmol) in H₂O (0.3 ml) were added to reaction mixture via syringe. The reaction mixture was stirred at 102°C for 3 more hours. After cooling down to room temperature, the reaction mixture was diluted with brine and extracted three times with ethyl acetate. The combined organic phases were dried over MgSO₄ and the solvent was removed under reduced pressure. Column chromatography (EtOAc/hexane 1:1 containing 1% NEt₃) gave the product in 46% yield (84 mg) as orange oil.

**¹H-NMR** (300 MHz, CDCl₃): 1.22-1.26 (t, ³*J* = 7.1, 3H, -CH₂-C**H₃**), 1.97-2.16 (m, 4H, -C**H₂**-CH₂-NBn₂ and H-15), 2.35 (s, 3H, -C**H₃**), 2.52-2.57 (t, ³*J* = 7.0, 2H, -C**H₂**-NBn₂), 2.69-2.74 (t, ³*J* = 7.4, 2H, -C**H₂**-CO₂Et), 2.80-2.85 (t, ³*J* = 7.6, 2H, -C**H₂**-CH₂-CH₂-NBn₂), 2.89-2.94 (t, ³*J* = 7.4, 2H, H-16), 3.07-3.13 (m, 4H, -C**H₂**-CH₂-CO₂Et and H-14), 3.59 (s, 4H, Bn-H), 4.10-4.17 (quartet, ³*J* = 7.1, 2H, -C**H**₂-CH₃), 6.93 (s, 1H, H-18), 7.19-7.38 (m,10H, Bn-**H**), 7.73-7.74 (d, ⁴*J* = 1.9,1H, H-4), 8.08-8.09 (s, ⁴*J* = 1.5, H-9), 8.63-8.64 (s, ⁴*J* = 1.9, 1H, H-2), 8.91-8.91 (s, ⁴*J* = 1.5, 1H, H-11).

**¹³C-NMR** (75,5 MHz, CDCl₃): δ 14.39 (-CH₂-**C**H₃), 19.34 (-**C**H₃), 25.70 (C-15), 27.53 (-**C**H₂-CH₂-NBn₂), 28.23 (**C**H₂-CH₂-CO₂Et), 32.32 (C-14), 33.02 (C-16), 34.82 (-**C**H₂-CO₂Et), 35.95 (-**C**H₂-CH₂-CH₂-NBn₂), 53.15 (-**C**H₂-NBn₂), 58.61 (Bn-C), 61.12 (-**C**H₂-CH₃), 116.73 (-CN), 118.92 (C-18), 126.95 (Bn-C), 128.35 (Bn-C), 129.03 (Bn-C), 131.46 (C-6), 131.96 (C-8), 135.49 (C-13), 136.71 (C-10), 138.02 (C-4), 138.83 (C-9), 140.04 (C-3), 140.16 (Bn-C), 140.92 (C-5), 147.32 (C-11), 149.63 (C-12), 150.73 (C-2), 152.19 (C-7), 156.05 (C-17), 160.63 (C-19), 171.96 (-**C**O₂Et).

| | | | |
|---|---|---|---|
| **HRMS:** | cal. 650.3490, exp. 650.3491 ([M+H]⁺) | | |
| **ESI-MS:** | cal. 650.3490, exp. 650.4 ([M+H]⁺) | | |
| **R_{f}(TLC)** | 0.25 (EtOAc:hexane 1:1 with 1% of NEt₃) | ***t*_{R}(HPLC)** | 6.11 min |

### Ethyl3-(5-(4-chloro-3-(3-(dibenzylamino)propyl)-6,7-dihydro-5H-cyclopenta[c]pyridine-1-yl)-2'-cyano-3-methyl-2,3'-bipyridin-5'-yl)propanoate (22c)

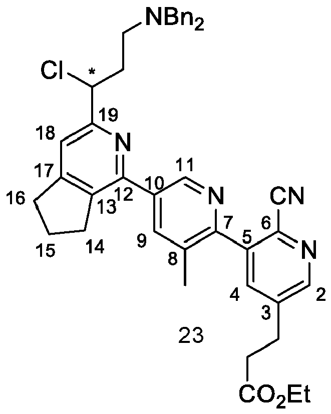

An oven-dried 20 ml microwave-flask equipped with magnetic stir bar was charged with **12b** (0.50 g, 1.3 mmol), potassium acetate (0.39 g, 4.0 mmol), bis(pinacolato)diboron (0.41 g, 1.6 mmol), tris(dibenzylideneacetone) dipalladium(0) (0.03 g, 0.034 mmol) and XPhos (0.13 g, 0.27 mmol) and was sealed with a microwave septum. The flask was evacuated and flushed with nitrogen. After addition of dioxane (12 ml), the reaction mixture was heated in the microwave to 130°C for 32 minutes. After cooling down to room temperature, **8I** (0.51 g, 1.2 mmol) in dioxane (3ml) and potassium phosphate (0.43 g, 2.0 mmol) in H₂O (1 ml) were added to reaction mixture via syringe. The reaction mixture was heated in the microwave to 100°C and stirred at this temperature for 55 minutes. After cooling down to room temperature, the reaction mixture was diluted with brine and extracted three times with ethyl acetate. The combined organic phases were dried over MgSO₄ and the solvent was removed under reduced pressure. Column chromatography (EtOAc/hexane 1:1) gave the product in 22% yield (180 mg) as orange oil.

**¹H-NMR** (300 MHz, CDCl₃): 1.22-1.26 (t, ³*J* = 7.1, 3H, -CH₂-C**H₃**), 2.11-2.34 (m, 7H, H-15, - CH₃, -CH₂-CH₂-NBn₂), 2.62-2.74 (m, 4H, -C**H₂**-CO₂Et, -C**H₂**-NBn₂), 2.95-3.00 (t, ³*J* = 7.5, 2H, H-16), 3.08-3.17 (m, 4H, -C**H₂**-CH₂-CO₂Et, H-14), 3.58 (s, 4H, Bn-H), 4.10-4.18 (quartet, ³*J* = 7.1, 2H, -C**H₂**-CH₃), 5.13-5.18 (t, ³*J* = 7.0 1H, -C**H**(Cl)-), 7.20-7.34 (m, 11H, Bn-H, H-18), 7.74-7.75 (d, ⁴*J* = 1.8, 1H, H-4), 8.09-8.10 (d, ⁴*J* = 1.5, 1H, H-9), 8.64-8.65 (s, ⁴*J* = 1.8, 1H, H-2), 8.93-8.93 (s, ⁴*J* = 1.5, 1H, H-11).

**¹³C-NMR** (75,5 MHz, CDCl₃): δ 14.38 (-CH₂-**C**H₃), 19.35 (-**C**H₃), 25.70 (C-15), 28.22 (-**C**H₂-CH₂-CO₂Et), 32.56 (C-14), 33.10 (C-16), 34.80 (-**C**H₂-CO₂Et), 35.99 (-**C**H₂-CH₂-NBn₂), 50.73 (-**C**H₂-NBn₂), 58.60 (Bn-C), 61.12 (-**C**H₂-CH₃), 62.06 (-**C**H(Cl)-), 116.71 (-CN), 118.06 (C-18), 127.06 (Bn-C), 128.37 (Bn-C), 129.05 (Bn-C), 131.43 (C-6), 131.97 (C-8), 136.05 (C-10), 137.69 (C-13), 137.99 (C-4), 138.89 (C-9), 139.62 (Bn-C), 140.07 (C-3), 140.80 (C-5), 147.29 (C-11), 149.49 (C-12), 150.78 (C-2), 152.46 (C-7), 156.92 (C-17), 158.40 (C-19), 171.96(-**C**O₂Et).

| | | | |
|---|---|---|---|
| **HRMS:** | cal. 684.3100, exp. 684.3067 ([M+H]⁺) | | |
| **ESI-MS:** | cal. 684.3495, exp. 684.3 ([M+H]⁺) | | |
| **R_{f}(TLC)** | 0.38 (EtOAc:hexane 4:6 with 2% MeOH) | *t*_{R}(HPLC) | 7.40 min |

### 3-[6-carbamoyl-5-[5-[3-[3-(dibenzylamino)propyl]-6,7-dihydro-5H-cyclopenta[c]pyridin-1-yl]-3,4-dimethyl-2-pyridyl]-3-pyridyl]propanoic acid (25a)

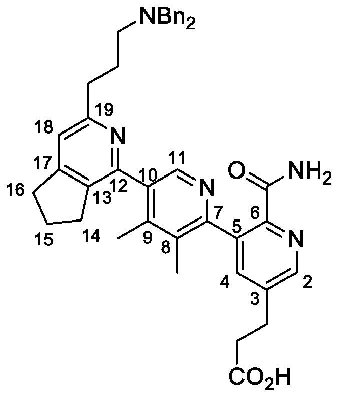

A 50 ml one-necked flask equipped with magnetic stir bar and condenser was charged was charged with **22a** (25 mg, 0.04 mmol) and of sodium hydroxide (15 mg, 0.4 mmol). After addition of ethanol (0.7 ml) and H₂O (0.3 ml), the reaction mixture was refluxed over night. After cooling down to room temperature, the reaction mixture was concentrated under reduced pressure. The product was purified by semipreparative HPLC (ACN/0.1 % TFA, H₂O/0.1% TFA) with the following gradient: 20% ACN to 99% ACN in H₂O in 60 minutes. **25a** eluted at t_{R} = 14.9 minutes and was obtained after lyophilisation as white ammonium trifluoroacetate salt in 73% yield (19 mg).

**¹H-NMR** (300 MHz, MeOD): 2.22-2.35 (m, 10H, -C**H₂**-CH₂-NBn₂, H-15, -C**H₃**), 2.76-2.81 (t, ³*J* = 7.2, 2H, -C**H₂**-CO₂H), 2.84-2.97 (m, 4H, H-16, -C**H₂**-NBn₂), 3.13-3.21 (m, 6H, H-14, -C**H₂**-CH₂-CH₂-NBn₂, -C**H₂**-CH₂-CO₂H), 4.41 (s, 4H, Bn-H), 7.47-7.55 (m, 11H, H-18, Bn-H), 7.92 (s, 1H, H-4), 8.49 (s, 1H, H-11), 8.78 (s, 1H, H-2).

**¹³C-NMR** (75,5 MHz, MeOD): δ 16.50 (-CH₃), 17.81 (-CH₃), 18.24 (C-15), 26.07 (-CH₂-CH₂-NBn₂), 28.85 (-**C**H₂-CH₂-CO₂H), 31.60, 33.69 (C-16, -C**H₂**-CH₂-CH₂-NBn₂), 34.67 (C-14), 35.36 (-**C**H₂-CO₂H), 52.38 (-**C**H₂-NBn₂), 58.57 (Bn-C), 122.23 (H-18), 130.68 (Bn-C), 130.74 (Bn-C), 131.49 (Bn-C), 132.46 (Bn-C), 134.31 (C-8), 135.12, (C-13), 140.92 (C-4), 141.15, 141.67, 141.85 (C-5, C-9, C-10), 142.60 (C-3), 143.90 (C-11), 145.58 (C-6), 151.10 (C-2), 151.91 (C-12), 155.55 (C-7), 157.98 (C-17), 162.39 (C-19), 167.20 (-**C**O₂H) 175.72 (-CH₂-CH₂-CO₂H).

| | | | |
|---|---|---|---|
| **HRMS:** | cal. 654.444, exp. 654.3275 ([M+H]⁺) | | |
| **ESI-MS:** | cal. 655.3444, exp. 654.2 ([M+H]⁺) | | |
| **m.p.** | 68.3°C | ***t*_{R}(HPLC)** | 4.22 min |

### 3-[6-carbamoyl-5-[5-[3-[3-(dibenzylamino)propyl]-6,7-dihydro-5H-cyclopenta[c]pyridin-1-yl]-3-methyl-2-pyridyl]-3-pyridyl]propanoic acid (25b)

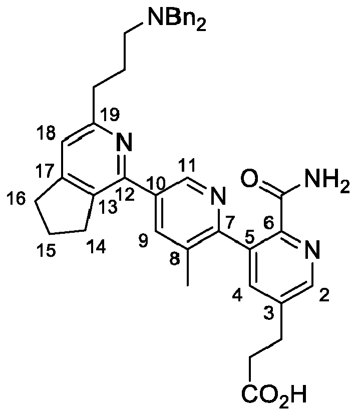

A 50 ml one-necked flask equipped with magnetic stir bar and condenser was charged was charged with **22b** (431 mg, 0.66 mmol) and sodium hydroxide (266 mg, 6.6 mmol). After addition of ethanol (20 ml) and H₂O (6 ml), the reaction mixture was refluxed over night. After cooling down to room temperature, the reaction mixture was diluted with water and extracted three times with diethyl ether. The aqueous phase was acidified to pH 1 with 1M HCl and extracted three times with ethyl acetate. The aqueous phase was then alkalized to pH 9 with saturated NaHCO₃ solution and extracted three times with n-butanol. The combined organic phases from pH 9 were dried over MgSO₄ and the solvent was removed under reduced pressure. The product was obtained as white solid in 80% yield (338 mg) and used without further purification in the next reaction step.

**¹H-NMR** (300 MHz, MeOD): 2.00-2.07 (m, 2H, -C**H₂**-CH₂-NBn₂),2.13-2.19 (m, 2H, H-15), 2.22 (s, 3H, -C**H₃**), 2.50-2.60 (m, 4H, -C**H₂**-NBn₂ and -C**H₂**-CO₂H), 2.80-2.84 (t, ³*J* = 7.2, 2H, - C**H₂**-CH₂-CH₂-NBn₂), 2.95-3.00 (t, ³*J* = 7.4, 2H, H-16), 3.08-3.16 (m, 4H, -C**H₂**-CH₂-CO₂H, H-14), 3.60 (s, 4H, Bn-H), 7.00 (s, 1H, H-18), 7.23-7.37 (m,10H, Bn-H), 7.73 (d, ⁴*J* = 1.9, 1H, H-4), 8.01 (s, 1H, H-9), 8.67 (s, 2H, H-2, H-11).

**¹³C-NMR (75,5** MHz, MeOD): δ 19.54 (-**C**H₃), 26.35 (C-15), 28.30 (-**C**H₂-CH₂-NBr)₂), 30.72 (-**C**H₂-CH₂-CO₂H), 33.02 (C-14), 33.94 (C-16), 36.24 (-CH₂-CH₂-CH₂-NBn₂), 40.10 (-**C**H₂-CO₂H), 53.65 (-**C**H₂-NBn₂), 59.63 (Bn-C) 120.13 (C-18), 128.07 (Bn-C), 129.45 (Bn-C), 130.27 (Bn-C), 133.31 (C-8), 136.28 (C-10), 137.11 (C-13), 139.18 (C-9), 140.15 (C-4), 141.11 (Bn-C), 142.77 (C-3), 144.90 (C-5), 146.22 (C-11), 147.03 (C-6), 150.03 (C-2), **151.33** (C-12), 157.96 (C-17), 158.79 (C-7), 161.62 (C-19), 169.76 (-**C**O₂H), 171.77 (-CH₂-CH₂-**C**O₂H).

The product was further purified by preparative HPLC (ACN/0.1 % TFA, H₂O/0.1% TFA) with the following gradient: 20% ACN to 99% ACN in H₂O in 50 minutes. **25b** eluted at t_{R} = 17.9 minutes and was obtained after lyophilisation as white ammonium trifluoroacetate salt.

**¹H-NMR** (300 MHz, MeOD): 2.31-2.37 (m, 7H, -C**H₂**-CH₂-NBn₂, H-15, -C**H₃**), 2.77-2.82 (t, ³*J* = 7.2, 2H, -C**H₂**-CO₂H), 2.93-2.97 (t, ³*J* = 7.1, 2H, -C**H₂**-NBn₂), 3.09-3.28 (m, 8H, H-14, H-16, - C**H₂**-CH₂-CH₂-NBn₂, -C**H₂**-CH₂-CO₂H), 4.43 (s, 4H, Bn-H), 7.40 (s, 1H, H-18), 7.40-7.47 (m,10H, Bn-H), 7.87-7.88 (d, ⁴*J* = 1.8, 1H, H-4), 8.47-8.47 (s, 1H, H-9), 8.80-8.80 (d, ⁴*J* = 1.8, 2H, H-2 or H-11), 8.85-8.86 (d, ⁴*J* = 1.4, 2H, H-2 or H-11).
**¹³C-NMR** (75,5 MHz, MeOD): δ 19.16, 24.31, 26.58, 28.81, 32.71, 34.13. 34.26, 35.49, 52.28, 58.58, 121.82, 130.65, 130.76, 131.47, 132.40, 135.65, 136.14, 139.57, 140.20, 141.77, 142.52, 142.99, 147.05, 148.27, 151.66, 156.72, 158.67, 161.67, 161.90, 168.68, 175.80.

| | | | |
|---|---|---|---|
| **HR-MS:** | **cal.** 640.3288, exp. 640.3294 ([M+H]⁺) | | |
| **ESI-MS:** | cal. 640.3288, exp. 640.3 ([M+H]⁺) | | |
| **m.p.** | 49.3 C | ***t*_{R}HPLC)** | 4.30 min |

### Example 3: Characterization of compounds

The first step of characterizing biologic activity of the compounds of the present invention as RIIBD mimetics was to test binding to RIIα. In table 1 is a list of the compounds selected for the first round of testing. In addition to **14b** several of its synthetic precursors were selected which have none or only some of the functional groups deprotected.

| **Designation** | **Compound** | **Designation** | **Compound** |
|---|---|---|---|
| **14b** | 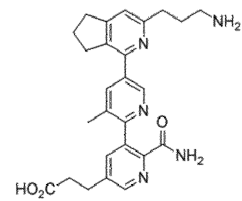 | **22a** | 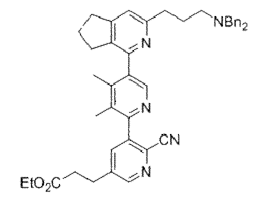 |
| **14c** | 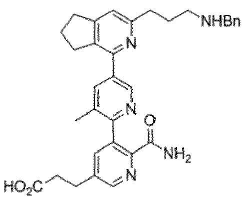 | **25a** | 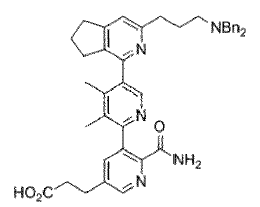 |
| **25b** | 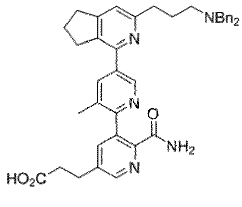 | **27** | 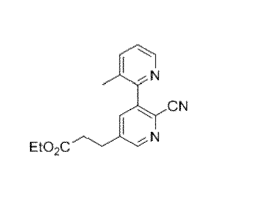 |

### Table 1: Compounds chosen for testing in STD- and HSQC-experiments.

The compounds were tested first in a saturation transfer difference (STD) NMR experiment. For the STD experiments with the compounds depicted in table 1 full length RIIα-His was used as the target protein. Prior to measurements of STD spectra in the presence of the target protein, the same set of experiments was performed in the absence of protein in order to confirm that no saturation of the compound signals takes place when irradiating the sample at the frequency chosen for protein irradiation. The results of the STD measurements are depicted in table 2. Compound 22a precipitated from solution.

**Table 2: Qualitative results from STD and HSQC experiments with the indicated compounds.**

| **compound** | **activity in STD** | **activity in HSQC** |
|---|---|---|
| **14b** | - | - |
| **14c** | - | - |
| **25b** | + | + |
| **22a** | not determined | not tested |
| **25a** | + | not tested |
| **27** | - | - |

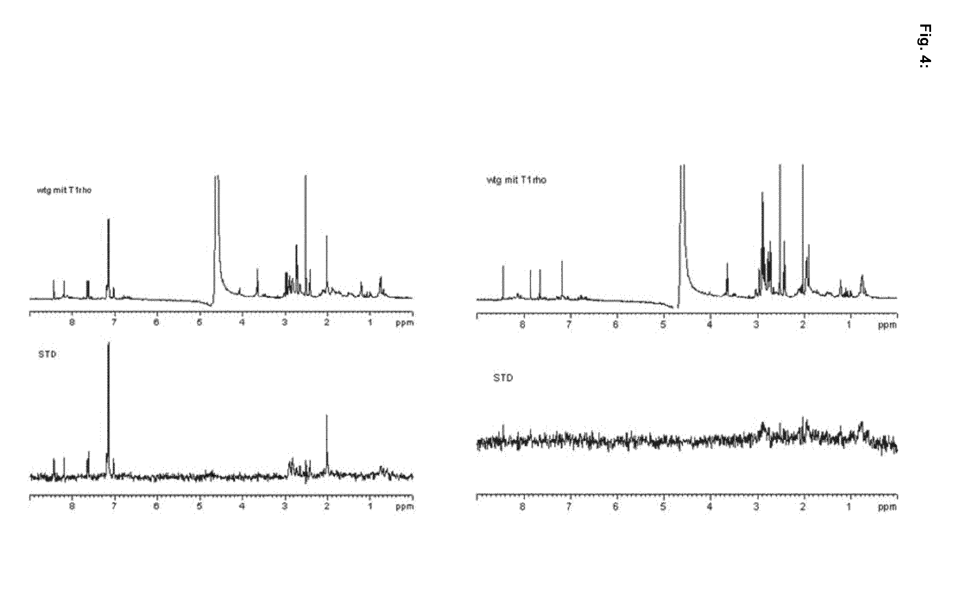

With the partially deprotected terpyridines **25a** and **25b** a reduction in ligand peak intensity was observed, indicating binding of the compounds to RIIα. No binding was detected in STD experiments with the deprotected terpyridine **14b**, partially deprotected terpyridine **14c** and bipyridine **27** which was obtained as by-product in the synthesis of **22b**. In Fig. 4, the STD-spectra of a binding compound (**25b**) and a non-binding compound (**14b**) are depicted. Fully protected terpyridine **22a** was tested in an STD experiment; however the compound crystallized from the solution and no ligand peaks were measurable by NMR.

For an estimate of binding specificity, the compounds were tested in a second NMR assay for binding to the D/D domain of RIIα. The D/D domain contains the AKAP binding site of RIIα. Therefore, suitable small molecule mimetics of the RIIBD of AKAP18 must bind to this domain of the full length protein. In other words, binding to the D/D domain is a good initial test for an AKAP-like, specific interaction of the compounds with RIIα.

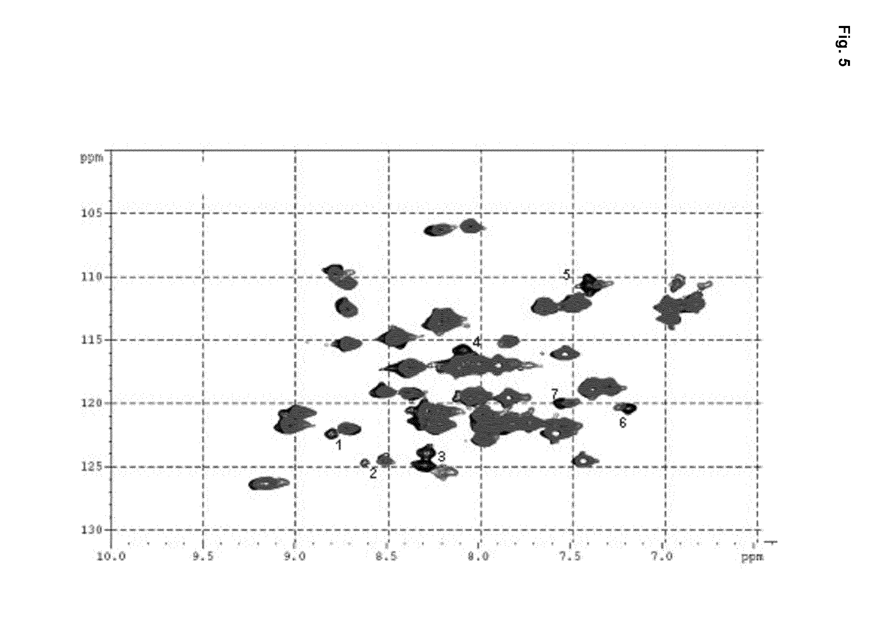

For this purpose HSQC-experiments with ¹⁵N-labeled D/D domain were employed with the compounds depicted in table 3. The results obtained correspond to the results from the STD experiments. A change in chemical shift of D/D domain signals was observed upon addition of terpyridine **25b**, while with the other compounds tested so far - terpyridines **14b**, **14c** and bipyridine **27 -** no perturbation of chemical shifts was seen. In Fig. 5, HSQC-spectra of the D/D domain in presence and absence of **25b** are depicted.

This outcome demonstrates that either presence of the *N,N*-dibenzylamine function is required for binding or that presence of a primary amine (**14b**) or secondary amine functionality (**14c**) in this position is detrimental for binding. Based on the nature of the binding site, which is described as a shallow hydrophobic grove, the inventors draw the conclusion that presence of the spacious, hydrophobic benzyl groups is important for mediating binding of the terpyridines to the D/D domain.

### Experimental Procedures

### Saturation transfer difference nuclear magnetic resonance (STD-NMR)

NMR samples for STD-NMR contained 0.1 mM of the indicated compound in NMR buffer, with a final concentration of d₆-DMSO of 2%. Samples were measured in presence or absence of 20 µM unlabelled PKA-RIIα FL(1-404)-His.

STD-NMR experiments were recorded with the carrier frequency set to -1000 (around -1 ppm) for on-resonance irradiation and about 330 ppm for off-resonance irradiation. A train of 50 Gaussian-shaped pulses at 40 ms was applied, each separated by a 1-ms delay, for a total duration of 2.05 s, to achieve selective protein saturation. Spectra acquisition was done with 32 scans in absence of protein and 256 scans in presence of protein and a relaxation delay of 1.3 s. A T_{1ρ} spin lock pulse of 40 ms was used to suppress the background protein signals. The STD spectrum was obtained from the internal subtraction of the on-resonance from the off-resonance data by phase cycling.

¹H,¹⁵N-Heteronuclear Single Quantum Coherence (HSQC)-NMR)

¹H,¹⁵N-HSQC-NMR experiments were carried on a 600 MHz spectrometer at 300 K.

¹H,¹⁵N-correlation experiments using with ¹⁵N-isotopically enriched RIIα(1-44) were recorded using a standard HSQC pulse program or an HMQC-SOFAST pulse program, both employing WATERGATE for solvent suppression.

The protein concentration employed for binding studies was in the order of 0.1 mM. The concentration of compounds was 0.3 mM for compounds and 0.8 mM for peptides, respectively. Experiments were performed in NMR buffer containing 5% d₆-DMSO. Each step was followed by recording of a new spectrum. The acquisition time in the direct dimension was restricted to 150 ms. A total of 256 increments was recorded in the indirect dimension.

## Claims

1. A compound **characterized by** a general formula I 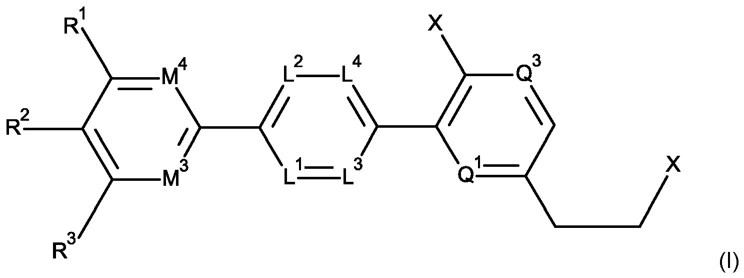 wherein
- R¹ is a moiety **characterized by** formula II 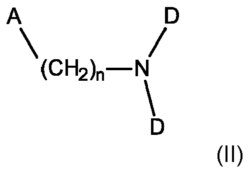 wherein
- A is oxygen, methylene, CHF, CHCl, CHBr, CHI, CF₂ or CCl₂, CFCl, n is 1, 2 or 3, each D independently is an aryl or heteroaryl, and the moiety is attached at A,
- R² is H or a moiety **characterized by** formula III 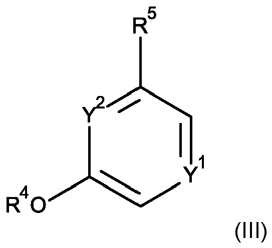 wherein
- R⁴ is H or methyl, R⁵ is a C₂-C₄ alkyl, Y¹ and Y² can be CH or N, and the moiety is attached at the C atom in the para-position to R⁵,
- M⁴ is N or CH,
- R³ is
- H or a C₁-C₄ alkyl and M³ is N or CH, or
- R³ and M³ form a cyclic alkyl or alkylether moiety,
- L¹ and L³ independently are CH, C-CH₃ or N, L² is N, CH or CH-COOH and L⁴, Q¹ and Q³ independently are CH or N, and
- each X independently is a CN, COOR⁶, or CONR⁶₂, wherein each R⁶ independently is H or C1-C4 alkyl,
- with the proviso that if either of M³ or M⁴ are N, L¹ and L² are not N, and if L³ or L⁴ is N, Q¹ is CH.

2. A compound according to claim 1, wherein the compound is **characterized by** a general formula IV 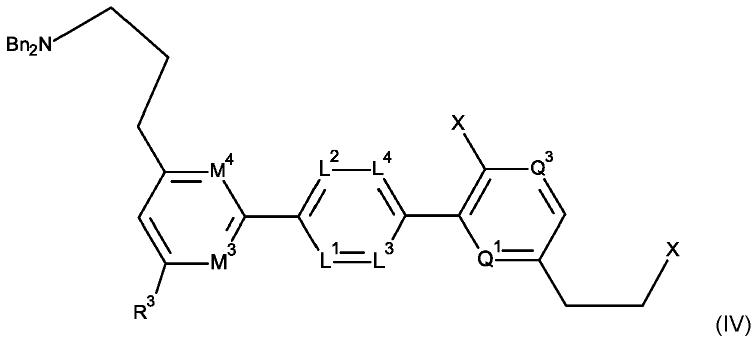 wherein
- R³ is H or a C₁-C₄ alkyl, M³ is N or CH, or R³ and M³ form a cyclic alkyl or alkylether moiety, M⁴ is N or CH,
- L¹ and L³ independently are CH, C-CH³ or N, L² is N, CH or CH-COOH and L⁴, Q¹ and Q³ independently are CH or N,
- each X independently is a CN, COOR⁶, or CONR⁶₂, wherein each R⁶ independently is H or C₁-C₄ alkyl,
- with the proviso that if either of M³ or M⁴ are N, L¹ and L² are not N, and if L³ or L⁴ is N, Q¹ is CH.

3. A compound according to claim 1 or 2, wherein the compound is **characterized by** a general formula V 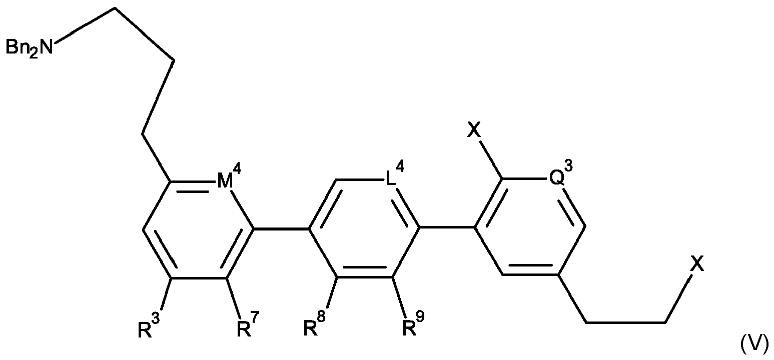 wherein
- M⁴, L⁴ and Q³ independently are CH or N,
- R³ and R⁷ independently are H or C₁-C₄-alkyl, or R³ and R⁷ form a cyclic alkyl or alkylether moiety,
- R⁸ and R⁹ independently are H or CH₃,
- each X independently can be a CN, COOR⁶, or CONR⁶₂, wherein each R⁶ independently is H or C₁-C₄ alkyl.

4. A compound according to one of the claims 1 or 3, wherein the compound is selected from the group consisting of
- 3-[6-carbamoyl-5-[5-[3-[3-(dibenzylamino)propyl]-6,7-dihydro-5H-cyclopenta[c]pyridin-1-yl]-3,4-dimethyl-2-pyridyl]-3-pyridyl]propanoic acid: 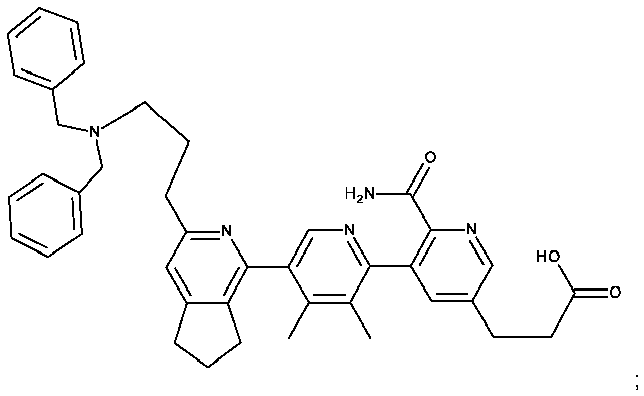
- 3-[6-carbamoyl-5-[5-[3-[3-(dibenzylamino)propyl]-6,7-dihydro-5H-cyclopenta[c]pyridin-1-yl]-3-methyl-2-pyridyl]-3-pyridyl]propanoic acid: 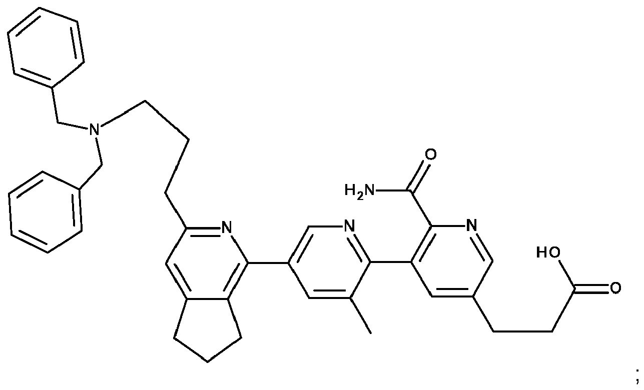
- alkyl 3-[6-cyano-5-[5-[3-[3-(dibenzylamino)propyl]-6,7-dihydro-5H-cyclopenta[c]pyridin-1-yl]-3,4-dimethyl-2-pyridyl]-3-pyridyl]propanoate: 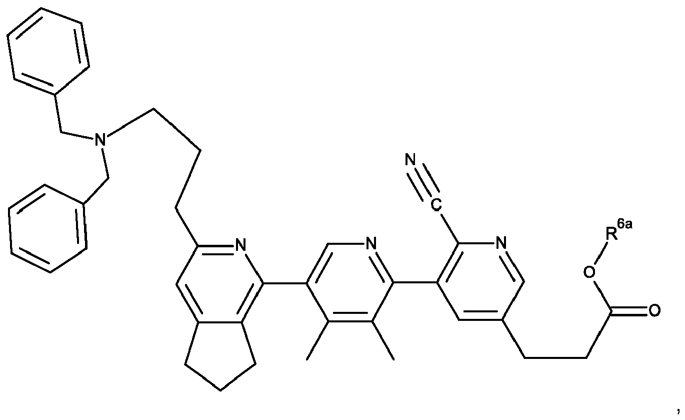 wherein
R^{6a} is C₁-C₄-alkyl, preferably ethyl;
- alkyl 3-[6-cyano-5-[5-[3-[3-(dibenzylamino)propyl]-6,7-dihydro-5H-cyclopenta[c]pyridin-1-yl]-3-methyl-2-pyridyl]-3-pyridyl]propanoate: 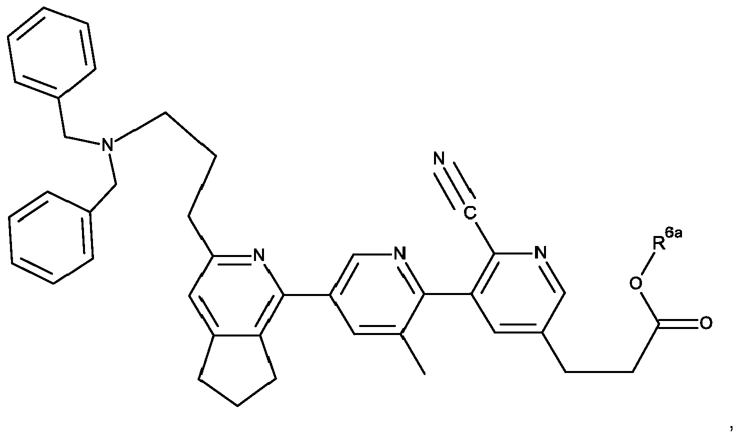 wherein
R^{6a} is C₁-C₄-alkyl, preferably ethyl;
- alkyl 3-[5-[5-[3-[1-chloro-3-(dibenzylamino)propyl]-6,7-dihydro-5H-cyclopenta[c]pyridin-1-yl]-3-methyl-2-pyridyl]-6-cyano-3-pyridyl]propanoate: 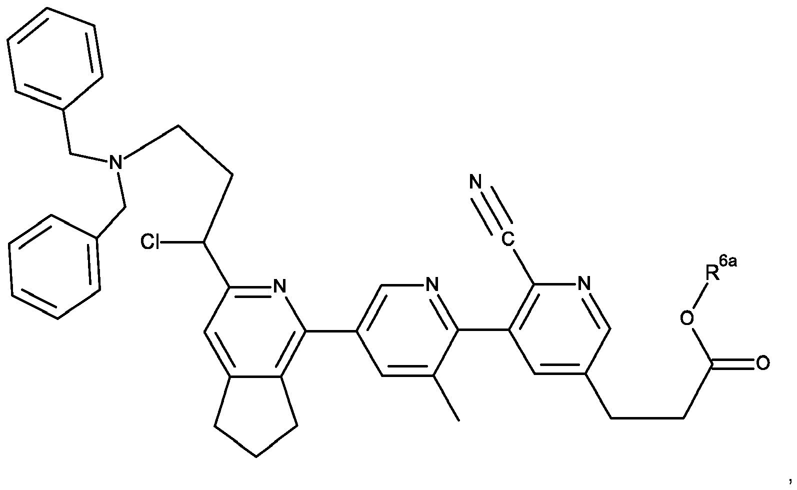 wherein
R^{6a} is C₁-C₄-alkyl, preferably ethyl;
- 3-[6-carbamoyl-5-[5-[4-(3-methoxy-5-propyl-2-pyridyl)-3-[2-(N-phenylanilino)ethoxy]phenyl]-3,4-dimethyl-2-pyridyl]-3-pyridyl]propanoic acid:

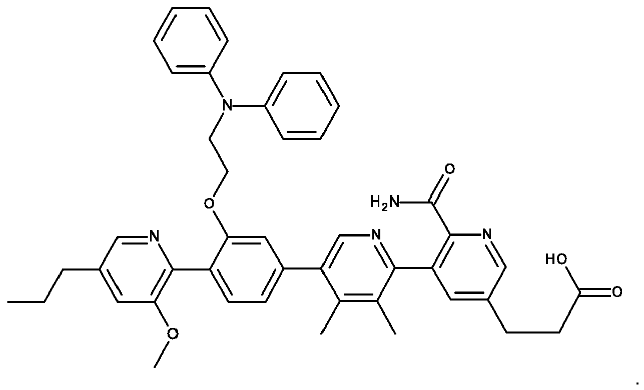

5. The compound:
- ethyl-3-[6-cyano-5-(4,4,5,5-tetramethyl-1,3,2-dioxaborolan-2-yl)pyridin-3-yl]propanoate,
- 3-(6,7-dihydro-5H-cyclopenta[c]pyridin-3-yl)propanenitrile,
- 3-(6,7-dihydro-5H-cyclopenta[c]pyridin-3-yl)propan-1-amine,
- *N,N*-dibenzyl-3-(6,7-dihydro-5H-cyclopenta[c]pyridin-3-yl)propan-1-amine,
- *N,N*-dibenzyl-3-(1-bromo-6,7-dihydro-5H-cyclopenta[c]pyridine-3-yl)propan-1-amine,
- *N,N*-dibenzyl-3-(1-chloro-6,7-dihydro-5H-cyclopenta[c]pyridine-3-yl)propan-1-amine,
- (rac)-*N,N*-dibenzyl-3-chloro-3-(1-chloro-6,7-dihydro-5H-cyclopenta[c]pyridine-3-yl)propan-1-amine,
- ethyl 3-(5-bromo-2'-cyano-3,4-dimethyl-2,3'-bipyridin-5'-yl)propanoate,
- ethyl 3-(5-bromo-2'-cyano-3-methyl-2,3'-bipyridin-5'-yl)propanoate.

6. The use of a compound according to one of the claims 1 to 4 as a medicament.

7. A pharmaceutical composition, comprising a compound according to one of claims 1 to 4.

8. A dosage form, comprising a compound according to one of claims 1 to 4.

9. A method of manufacture a medicament, comprising the use of a compound according to one of claims 1 to 4.

10. A method for prevention or treatment of heart failure, comprising the administration of a compound according to one of claims 1 to 4 to a patient in need thereof.
